# EUROPEAN PATENT APPLICATION

(11) **EP 4 442 257 A1**
(43) Date of publication of application: **09.10.2024**
(21) Application number: 23166585.2
(22) Date of filing: 04.04.2023
(51) Int. Cl.: A61K 9/51, C12N 15/11

(54) **NANOPARTICLE COMPOSITIONS COMPRISING ONE OR MORE VARIANTS OF POLY(D,L-LACTIC-CO-GLYCOLIC ACID)**

(71) Applicant: Københavns Universitet, 2200 København N (DK)
(72) Inventor: FOGED, Camilla, 2200 København N (DK); LOKRAS, Abhijeet Girish, 2200 København N (DK); THAKUR, Aneesh, 2200 København N (DK); FRANZYK, Henrik, 2200 København N (DK); BAGHEL, Saahil Sandeep, 2200 København N (DK)
(74) Representative: Plougmann Vingtoft a/s

(57) **Abstract**

The present invention has been made within the field of nanoparticles and relates to a nanoparticle composition comprising the polymer poly(D,L-lactic-co-glycolic acid) (PLGA). In particular the present invention relates to a lipid polymer hybrid nanoparticle composition comprising a cationic or cationically ionisable lipid or lipid-like material, a helper lipid, a lipopolymer, and one or more variants of PLGA. The nanoparticle composition is particularly useful for delivery of nucleic acid molecules, specifically mRNA; thereby making them highly suitable for use in vaccines, such as for the prevention and/or treatment of infectious diseases.

## Description

### Technical field of the invention

The present invention has been made within the field of nanoparticles and relates to a nanoparticle composition comprising the polymer poly(D,L-lactic-co-glycolic acid) (PLGA). In particular, the present invention relates to a lipid polymer hybrid nanoparticle composition comprising a cationic or cationically ionisable lipid or lipid-like material, a helper lipid, a lipopolymer, and one or more variants of PLGA. The nanoparticle composition is particularly useful for delivery of nucleic acid molecules, specifically mRNA; thereby making them highly suitable for use in vaccines, such as for the prevention and/or treatment of infectious diseases.

### Background of the invention

Vaccines based on messenger RNA (mRNA) represent the beginning of a possible paradigm shift in the prevention of diseases of global significance, *e.g*., viral infections. Lipid nanoparticles (LNPs) are commonly used as a delivery system to protect mRNA against degradation, and enable intracellular delivery of mRNA, but LNPs are characterised by low colloidal stability and high shear stress sensitivity, which challenges their handling, storage, and administration. For example, when handling mRNA vaccines, physical stressors like shaking should be avoided, because shaking has been shown to cause loss of vaccine potency. In addition, processing of liquid formulations based on mRNA-loaded LNPs into solid dosage forms using for example spray drying is challenged by shear forces imposed on the LNPs during the drying process, eventually causing drug leakage and loss of mRNA activity. The shear stress imposed on mRNA-loaded LNP formulations for pulmonary delivery by the nebulisation process has also been shown to cause particle aggregation and mRNA leakage.

Hybrid nanoparticles composed of lipids and polymers are promising alternatives to LNPs for mRNA delivery. Lipid-polymer hybrid nanoparticles (LPNs) are composed of one or several lipid components, for example an ionisable or cationic lipid or lipid-like material that is used for complexation of nucleic acid-based cargos, stabilising non-ionic lipids, e.g., PEG-lipids, and a hydrophobic helper polymer, which stabilises the LPN structure. The colloidal stability in liquid formulations and in biological media is higher for LPNs than for LNPs, probably due to hydrophobic interactions in the LPNs between the lipid and the polymer. This higher colloidal stability might enable post-manufacture/downstream processing of liquid LPN formulations, for example spray drying into dry powder inhaler formulations for pulmonary delivery. The hydrophobic helper polymer used in LPNs might be the matrix-forming and biodegradable polymer poly(D,L-lactic-co-glycolic acid) (PLGA).

WO 2017/158093 A1 relates to a nanoparticle composition comprising PLGA derivatives and a lipid. Said nanoparticles are suitable for medical use. The PLGA nanoparticles may comprise one or more lipids represented by a Markush structure in claim 1. Thus, the lipids used in WO 2017/158093 A1 to develop PLGA nanoparticles are all rather similar and the resulting PLGA nanoparticles might therefore lack crucial components such as stabilising helper lipids or a lipopolymer. In fact, the inventors of the present invention have shown that LPNs composed only of ionisable cationic lipid (98N12-5 (also known as L₅N₁₂) or C12-200) and PLGA did not mediate functional cellular delivery of mRNA to the cytosol, neither *in vitro* nor *in vivo* (results not shown). In subsequent pilot experiments, the inventors found that inclusion of two additional phospholipids in the LPNs is essential for mRNA transfection and colloidal stabilisation.

Zhao, W. *et al.* disclose a lipid polymer hybrid nanomaterial for mRNA delivery. The inventors had previously developed an N¹,N³,N⁵-tris(2-aminoethyl)benzene-1,3,5-tricarboxamide (TT) derived lipid-like nanomaterial (TT3-LLN), which was capable of effectively delivering multiple types of mRNA. The inventors optimised the nanomaterial by incorporating different polymers. The lipid polymer hybrid nanomaterial comprises TT3 lipid, 1,2-dioleoyl-*sn*-glycero-3-phosphoethanolamine (DOPE), cholesterol, 1,2-dimyristoyl-*rac*-glycero-3-methoxypolyethylene glycol-2000 (DMG-PEG₂₀₀₀) and different polymers. The different polymers used by Zhao, W. *et al.* are listed in table 1, wherein four out of seven polymers are derivatives of PLGA (PLGA1, PLGA2, PLGA3, and PLGA4). Since the inventors use cholesterol, the resulting LPNs might simply be LNPs and would therefore not have the improved colloidal stability characteristic for the LPNs as compared to regular LNPs. Hence, an improved nanoparticle design would be advantageous, and in particular, nanoparticles with a high colloidal stability would be advantageous.

### Summary of the invention

Thus, an object of the present invention relates to a nanoparticle composition with a high colloidal stability. In particular, it is an object of the present invention to provide a nanoparticle composition that solves the above-mentioned problems of the prior art by providing a nanoparticle composition that is colloidal stable and displays an efficient intracellular delivery of nucleic acids.

Thus, one aspect of the invention relates to a nanoparticle composition comprising a cationic or cationically ionisable lipid or lipid-like material, a helper lipid, a lipopolymer, and one or more variants of poly(D,L-lactic-*co*-glycolic acid) (PLGA), wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9‴′,9‴ʺ- ((((benzene-1,3,5-tricarbonyl)yris(azanediyl)) tris (propane-3,1-diyl)) tris(azanetriyl))hexanonanoate (FTT5), 1,2-dioleoyl-3-trimethylamonniumpropane (DOTAP), dimethyldioctadecylammonium bromide (DDAB), 1,2-dioleoyl-3-dimethylammonium-chloride (DODAC), and 1,2-di-O-octadecenyl-3-trimethylammonium propane (DOTMA), or any mixture thereof, wherein the helper lipid is selected from the group consisting of 1,2-dioleoyl-*sn-*glycero-3-phosphoethanolamine (DOPE), 1,2-dioleoyl-sn-glycero-3-phosphocholine (DOPC), 1,2-dipalmitoyl-*sn*-glycero-3-phosphocholine (DPPC), 1,2-distearoyl-*sn*-glycero-3-phosphocholine (DSPC), and 1,2-ditetradecanoyl-*sn-*glycero-3-phosphocholine (DMPC), or any mixture thereof, and wherein the lipopolymer is a polyethylene glycol (PEG)- or polysarcosine-lipid conjugate, or a PEG- or polysarcosine-lipid like conjugate, or any mixture thereof.

Another aspect relates to a vaccine composition comprising the nanoparticle composition according to any one of the preceding claims and at least one nucleic acid encoding an antigen. Yet another aspect relates to said vaccine composition for use in the prevention and/or treatment of an infectious disease.

In addition, another aspect relates to a process for obtaining the nanoparticle composition according to the present invention, said process comprising the steps of:
a) Providing an cationic or cationically ionisable lipid or lipid-like material, a helper lipid, a lipopolymer, one or more variants of poly(D,L-lactic-co-glycolic acid) (PLGA), and at least one nucleic acid;
b) Dissolving the cationic or cationically ionisable lipid or lipid-like material, the helper lipid, the lipopolymer, and the one or more variants of PLGA of step a) in an organic solvent comprising acetonitrile supplemented with acetic acid, thereby providing an organic phase;
c) Diluting the at least one nucleic acid of step a) in an aqueous solvent comprising a buffer with a pH within the range of 3.0 to 7.8, thereby providing an aqueous phase;
d) Mixing the organic phase of step b) with the aqueous phase of step c) to obtain nanoparticles by nanoprecipitation;
e) Performing filtration, preferably tangential flow filtration or dialysis, of the nanoparticles of step d) to obtain a nanoparticle composition;

wherein the cationic or cationically ionisable lipid or lipid-like material is selected from the group consisting of 1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), dilinoleylmethyl-4-dimethylaminobutyrate (DLin-MC3-DMA), N1,N3,N5-tris(3-(didodecylamino)propyl)benzene-1,3,5-tricarboxamide (TT3), 2,2-dilinoleyl-4-dimethylaminoethyl-[1,3]-dioxolane (DLin-KC2-DMA), hexa(octan-3-yl) 9,9',9",9‴,9‴′,9‴ʺ- ((((benzene-1,3,5-tricarbonyl)yris(azanediyl)) tris (propane-3,1-diyl)) tris(azanetriyl))hexanonanoate (FTT5), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), DOPC, DOPG, DPPC, DSPC, DPPG, DMPC, MGDG, DGDG, and SQDG, or any mixture thereof, and
wherein the lipopolymer is a PEG- or polysarcosine-lipid conjugate, or a PEG- or polysarcosine-lipid like conjugate, or any mixture thereof.

Another aspect relates to a nanoparticle composition obtained using the process of the present invention.

### Brief description of the figures

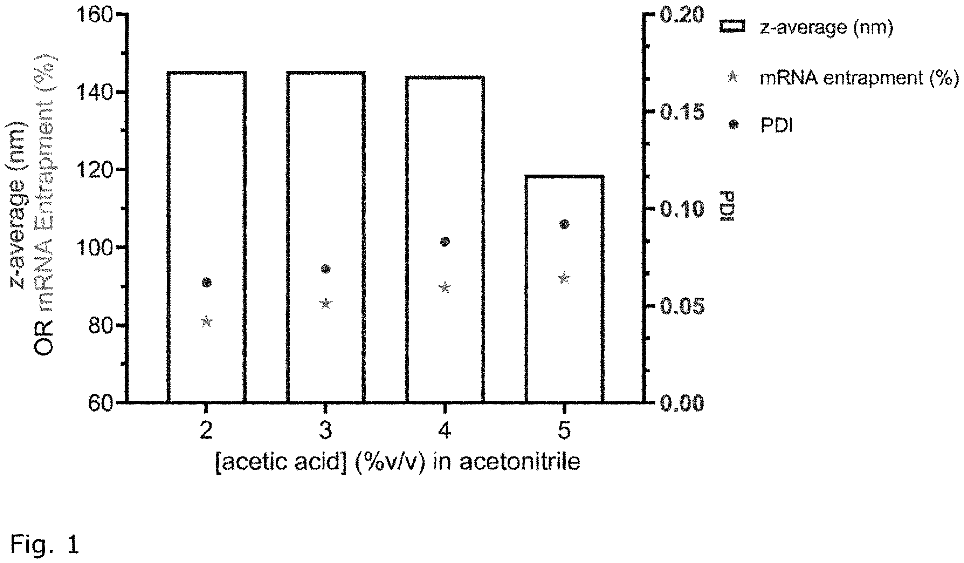

**Figure 1** shows the z-average, mRNA entrapment, and PDI of LPNs prepared using acetonitrile supplemented with 2 to 5 % (v/v) acetic acid. Increasing the concentration of glacial acetic acid in acetonitrile from 2 to 5 % (v/v) results in approximately 12% increase in *FLuc* mRNA entrapment efficiency (bars represent mean values, N = 2).

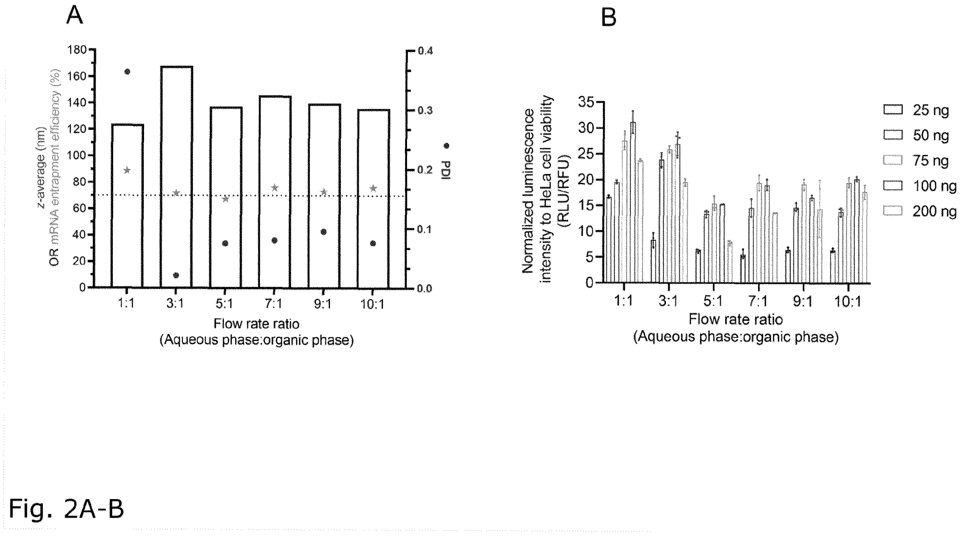

**Figure 2A** shows the z-average, mRNA entrapment, and PDI of *FLuc* mRNA-loaded LPNs prepared using different flow rate ratios (N = 1). The dotted line represents 70 units on the left Y axis. The *FLuc* mRNA-loaded LPNs (C12-200:FLuc mRNA ratio 10:1, total lipid content 50% w/w, 7 mol% DMPE-PEG₂₀₀₀) were prepared using the NanoAssemblr^{®} Benchtop^{™} microfluidic mixer.
**Figure 2B** shows the luminescence intensity normalised to cell viability of HeLa cells incubated for 24 h with five different mRNA doses (25, 50, 75, 100, and 200 ng) of *FLuc* mRNA-loaded LPNs prepared at six different FRRs (1:1, 3:1, 5:1, 7:1, 9:1, and 10:1). Bars represent mean values ± SD, n = 4 technical replicates. The *FLuc* mRNA-loaded LPNs (C12-200:*FLuc* mRNA ratio 10:1, total lipid content 50% w/w, 7 mol% DMPE-PEG₂₀₀₀) were prepared using the NanoAssemblr^{®} Benchtop^{™} microfluidic mixer.

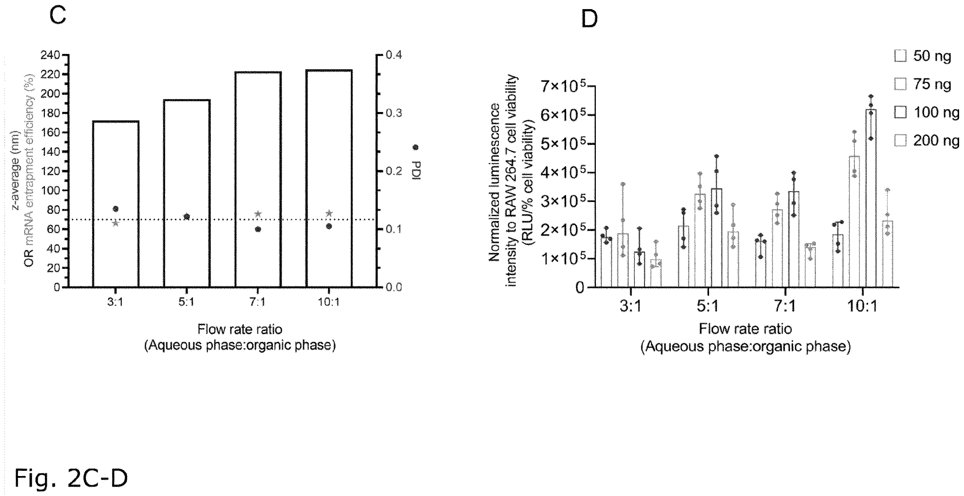

**Figure 2C** shows the *z*-average, mRNA entrapment, and PDI of *FLuc* mRNA-loaded LPNs prepared using different flow rate ratios. Data points represent mean values of two biological replicates (N = 2). The *FLuc* mRNA-loaded LPNs (RAW 264.7 cells, C12-200:*FLuc* mRNA ratio 10:1, total lipid content 50% w/w, 2.5 mol% DMG-PEG₂₀₀₀) were prepared using the NanoAssemblr^{®} Ignite^{™} microfluidic mixer.
**Figure 2D** shows the normalised luminescence intensity to cell viability of RAW 264.7 murine macrophages incubated for 24 h with four different mRNA doses (50, 75, 100, and 200 ng) of *FLuc* mRNA-loaded LPNs prepared at four different FRRs (3:1, 5:1, 7:1, and 10:1). Bars represent mean values ± SD, N = 2, n = 8 technical replicates. The *FLuc* mRNA-loaded LPNs (C12-200:FLuc mRNA ratio 10:1, total lipid content 50% w/w, 7 mol% DMPE-PEG₂₀₀₀) were prepared using the NanoAssemblr^{®} Benchtop^{™} microfluidic mixer.

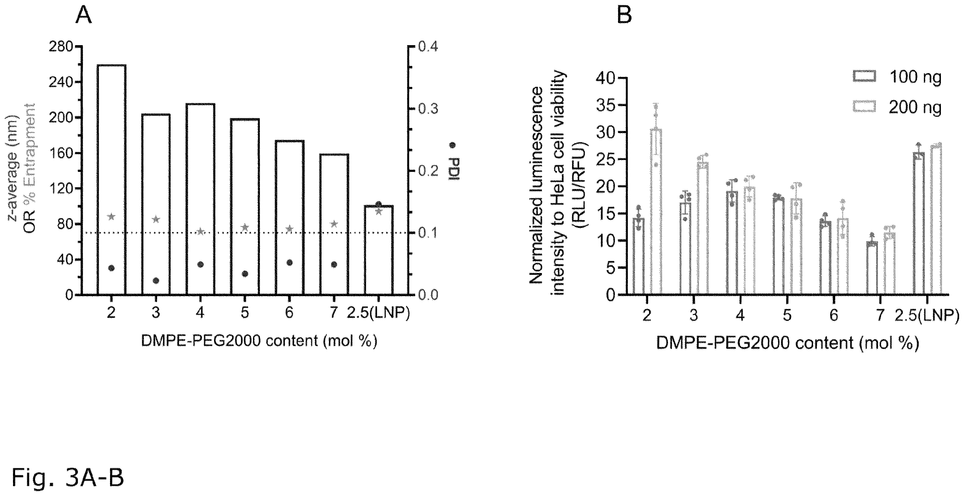

**Figure 3A** shows the z-average, mRNA entrapment, and PDI of *FLuc* mRNA-loaded LPNs prepared at six different molar percentages of DMPE-PEG₂₀₀₀ (2, 3, 4, 5, 6, and 7 mol%). For comparison, LNPs with 2.5 mol% DMPE-PEG₂₀₀₀ are shown (N = 1). The dotted line represents 70 units on the left Y axis.
**Figure 3B** shows the normalised luminescence intensity to cell viability of HeLa cells incubated for 24 h with two different mRNA doses (100 and 200 ng) of *FLuc* mRNA-loaded LPNs, prepared at six molar ratios of DMPE-PEG₂₀₀₀ (2, 3, 4, 5, 6, and 7 mol%). For comparison, LNPs with 2.5 mol% DMPE-PEG₂₀₀₀ are shown. Bars represent mean values ± SD, n = 4 technical replicates.

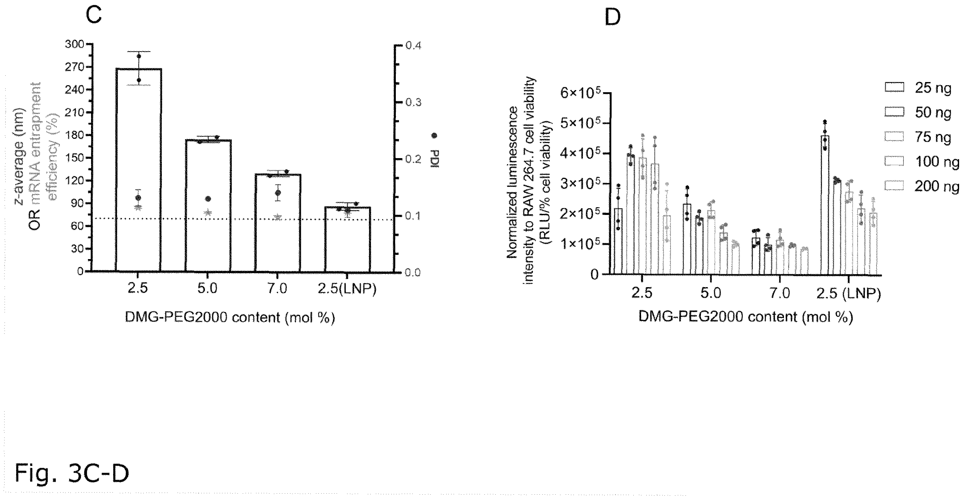

**Figure 3C** shows the z-average, mRNA entrapment, and PDI of *FLuc* mRNA-loaded LPNs prepared at three different molar percentages of DMG-PEG₂₀₀₀ (2.5, 5, and 7 mol%) (N = 2). For comparison, LNPs with 2.5 mol% DMG-PEG₂₀₀₀ are shown (N = 2). Data points represent mean values of two biological replicates (N = 2).
**Figure 3D** shows the normalised luminescence intensity to cell viability of RAW 264.7 murine macrophages incubated for 24 h with different five different mRNA doses (25, 50, 75, 100, and 200 ng) of *FLuc* mRNA-loaded LPNs prepared at three different molar percentages of DMG-PEG₂₀₀₀ (2.5, 5, and 7 mol%) (N = 2). Bars represent mean values ± SD, N = 2, n = 8 technical replicates.

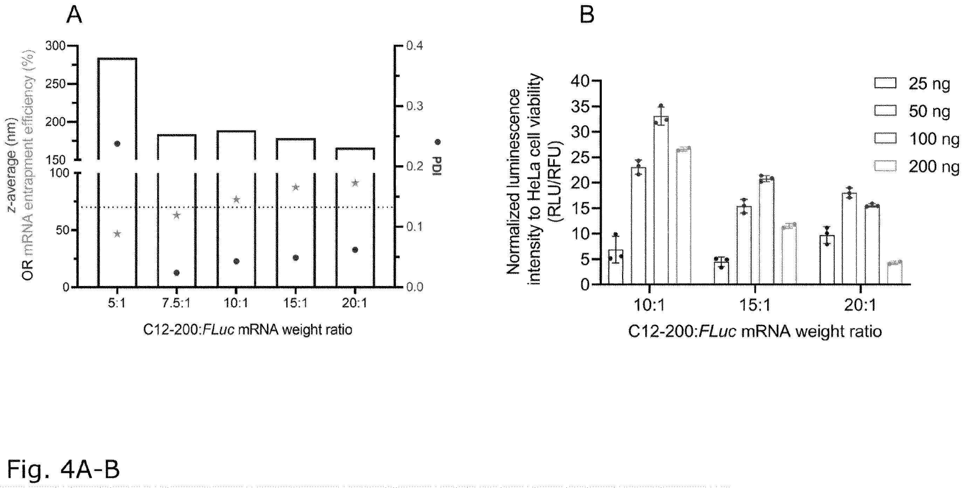

**Figure 4A** shows the z-average, mRNA entrapment, and PDI of *FLuc* mRNA-loaded LPNs prepared at five different C12-200:*FLuc* mRNA weight ratios (5:1, 7.5:1, 10:1, 15:1, and 20:1) (N = 1). The dotted line represents 70 units on the left Y axis. The *FLuc* mRNA-loaded LPNs (DMPE-PEG₂₀₀₀ content 7 mol%, total lipid content 50% w/w, FRR 3:1) were prepared by using the NanoAssemblr^{®} Benchtop microfluidic mixer.
**Figure 4B** shows the normalised luminescence intensity to cell viability of HeLa cells incubated for 24 h with four different mRNA doses (25, 50, 100, and 200 ng) of *FLuc* mRNA-loaded LPNs prepared at three different C12-200:*FLuc* mRNA weight ratios (10:1, 15:1, and 20:1). Bars represent mean values ± SD (n = up to 4 technical replicates). The *FLuc* mRNA-loaded LPNs (DMPE-PEG₂₀₀₀ content 7 mol%, total lipid content 50% w/w, FRR 3:1) were prepared by using the NanoAssemblr^{®} Benchtop microfluidic mixer.

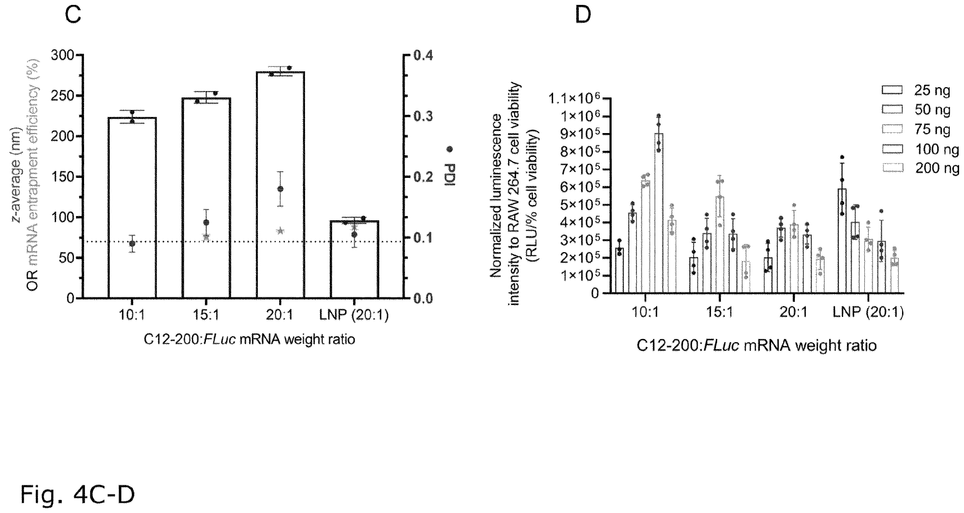

**Figure 4C** shows the z-average, mRNA entrapment and PDI of *FLuc* mRNA-loaded LPNs prepared at three different C12-200:*FLuc* mRNA weight ratios (10:1, 15:1, and 20:1) (N = 1). For comparison, LNPs prepared at a C12-200:*FLuc* mRNA weight ratio of 20:1 are shown. The dotted line represents 70 units on the left Y axis. The FLuc mRNA-loaded LPNs (DMG-PEG₂₀₀₀ content 2.5 mol%, total lipid content 50% w/w, FRR 10:1) were prepared using the NanoAssemblr^{®} Ignite^{™} microfluidic mixer.
**Figure 4D** shows the normalised luminescence intensity to cell viability of RAW 264.7 cells incubated for 24 h with five different mRNA doses (25, 50, 75, 100, and 200 ng) of *FLuc* mRNA-loaded LPNs prepared at three different C12-200:FLuc mRNA weight ratios (10:1, 15:1, and 20:1). For comparison, LNPs prepared at a *C12-200:FLuc* mRNA weight ratio of 20:1 are shown. The *FLuc* mRNA-loaded LPNs (DMG-PEG₂₀₀₀ content 2.5 mol%, total lipid content 50% w/w, FRR 10:1) were prepared using the NanoAssemblr^{®} Ignite^{™} microfluidic mixer.

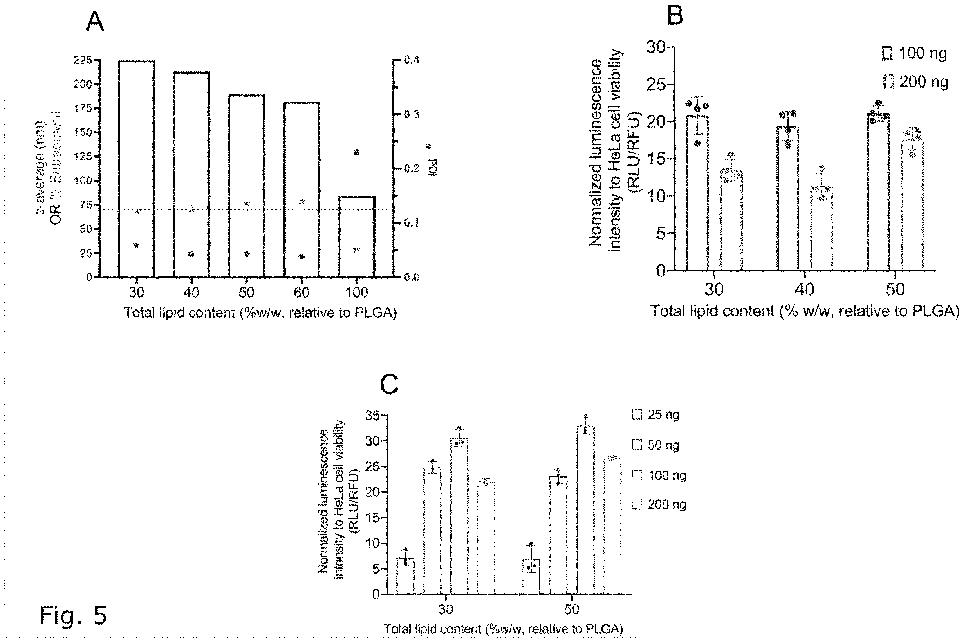

**Figure 5A** shows the z-average, mRNA entrapment and PDI of *FLuc* mRNA-loaded LPNs (C12-200:*FLuc* mRNA ratio 10:1, DMPE-PEG₂₀₀₀ content 7 mol%, FRR 3:1) prepared at five different total lipid contents (30, 40, 50, 60 and 100%).
**Figure 5B** shows the normalised luminescence intensity to cell viability of HeLa cells incubated for 24 h with two different mRNA doses (100, and 200 ng) of *FLuc* mRNA-loaded LPNs (C12-200:*FLuc* mRNA ratio 10:1, DMPE-PEG₂₀₀₀ content 7 mol%, FRR 3:1) prepared at three different total lipid contents (30, 40, and 50%). Bars represent mean values ± SD, n = 4 technical replicates.
**Figure 5C** shows the normalised luminescence intensity to cell viability of HeLa cells incubated for 24 h with four different mRNA doses (25, 50, 100, and 200 ng) of *FLuc* mRNA-loaded LPNs (C12-200:*FLuc* mRNA ratio 10:1, DMPE-PEG₂₀₀₀ content 7 mol%, FRR 3:1) prepared at two different total lipid contents (30 and 50%). Bars represent mean values ± SD, n = 3 technical replicates.

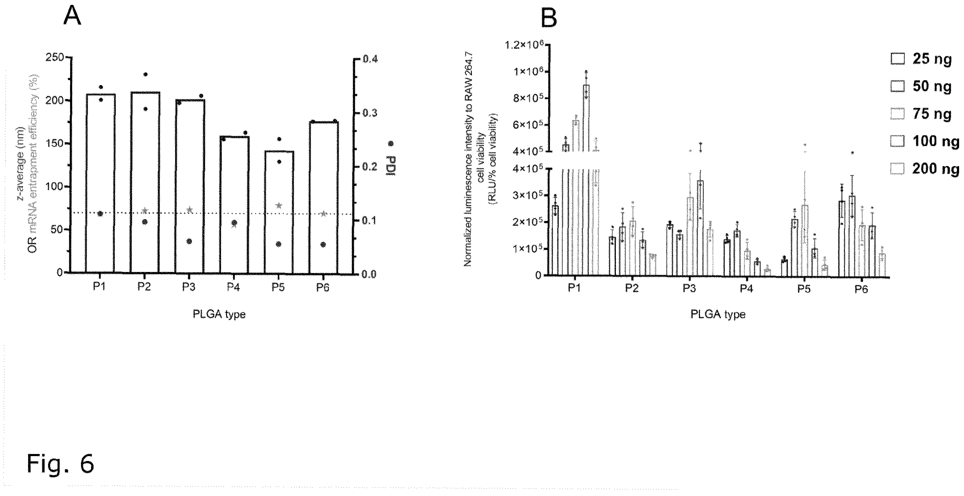

**Figure 6A** shows the z-average, mRNA entrapment and PDI of *FLuc* mRNA-loaded LPNs, prepared using six different PLGA types (P1, P2, P3, P4, P5, and P6, see Table 1 for code explanation) (N = 2).
**Figure 6B** shows the normalised luminescence intensity to cell viability of RAW 264.7 cells incubated for 24 h with five different mRNA doses (25, 50, 75, 100, and 200 ng) of *FLuc* mRNA-loaded LPNs, prepared using six different PLGA types (P1, P2, P3, P4, P5, and P6, see Table 1 for code explanation) (N = 2). Bars represent mean values ± SD, N = 2 biological replicates, n = up to 8 technical replicates.

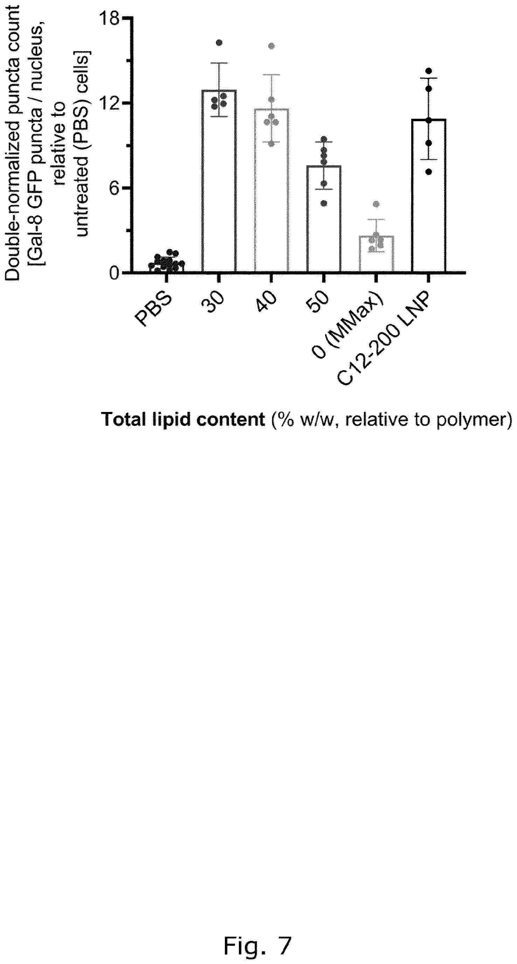

**Figure 7** shows the double-normalised puncta count [Gal-8 GFP puncta/nucleus, relative to untreated cells (PBS)] of HEK 293T/17 Gal8-GFP reporter cells incubated for 24 h with an mRNA dose of 200 ng of *FLuc* mRNA-loaded LPNs prepared at four different total lipid contents (30, 40, and 50%) at a C12-*200:FLuc* mRNA weight ratio of 10:1. For comparison, cells transfected with Lipofectamine^{®} MessengerMAX^{™} (MMax) and control C12-200 LNPs are shown. Bars represent mean values ± SD, each dot represents one image from two wells per formulation.

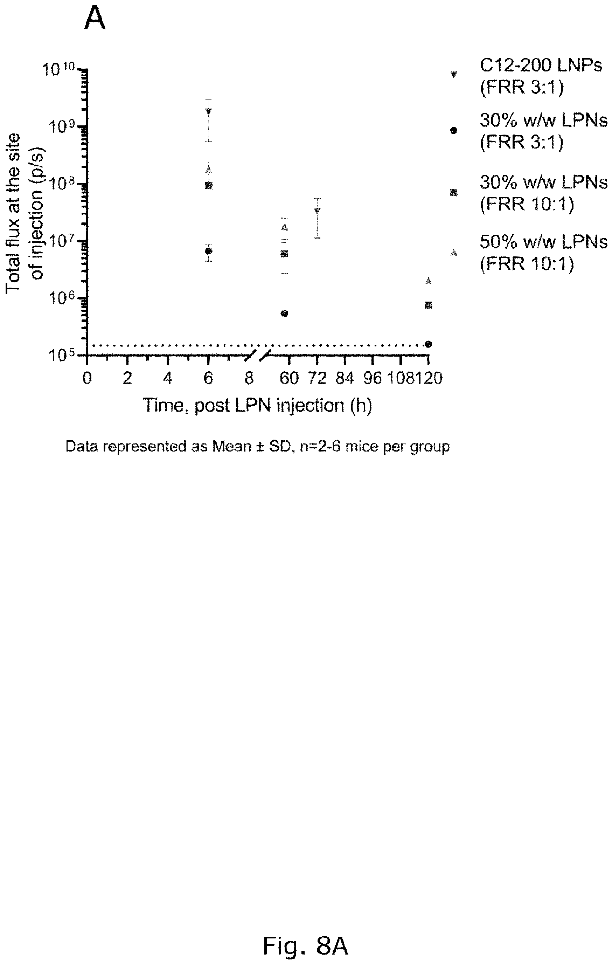

**Figure 8A** shows the total flux at the site of injection for mice injected with *FLuc* mRNA-loaded LPNs (7 mol% DMPE-PEG₂₀₀₀, C12-200:mRNA ratio 10:1) prepared at two different FRRs (3:1 and 10:1) at two different total lipid contents (30% and 50%).

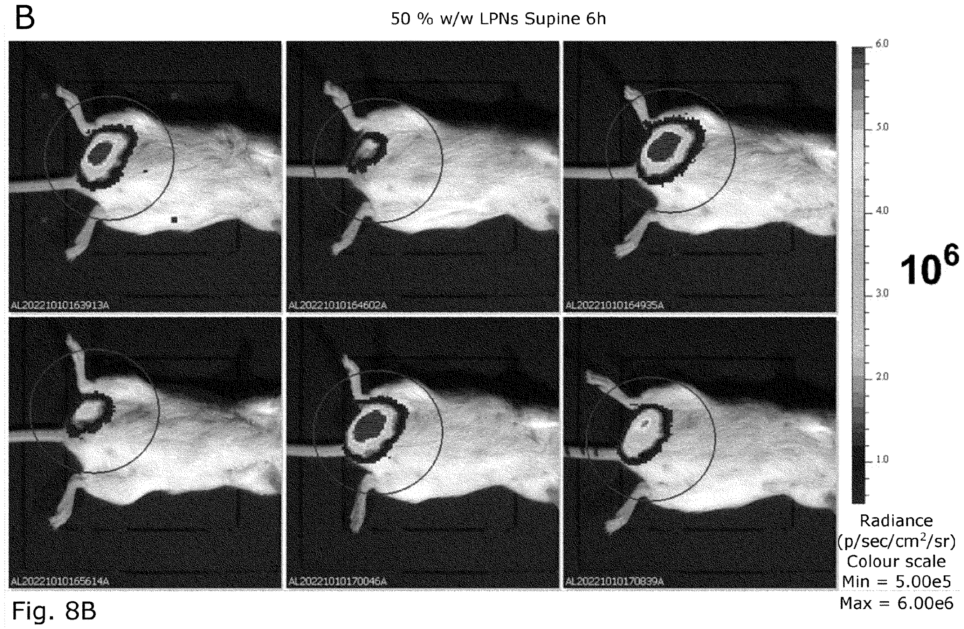

**Figure 8B** shows representative images of mice dosed with 50% (w/w) LPNs captured from the supine position 6 h post injection.

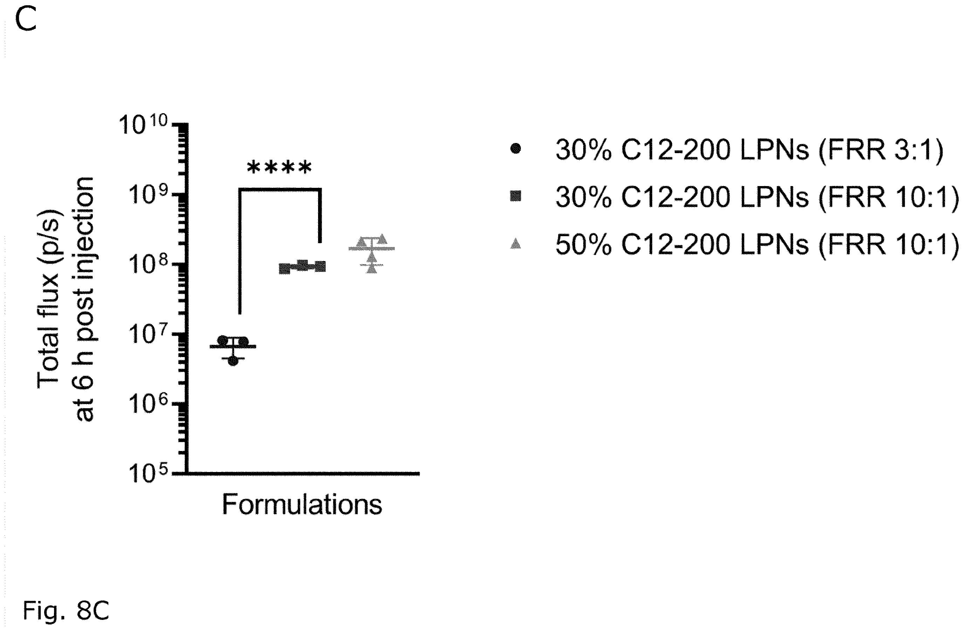

**Figure 8C** shows the total luminescence flux at the site of injection 6 h post injection. Means of data points ± SD are given, n = 3-6 mice per group. Statistically significant differences are shown using two-tailed, unpaired t-test. *p* values: ^{∗}*p* < 0.05, and ^{∗∗∗∗}*p* < 0.0001.

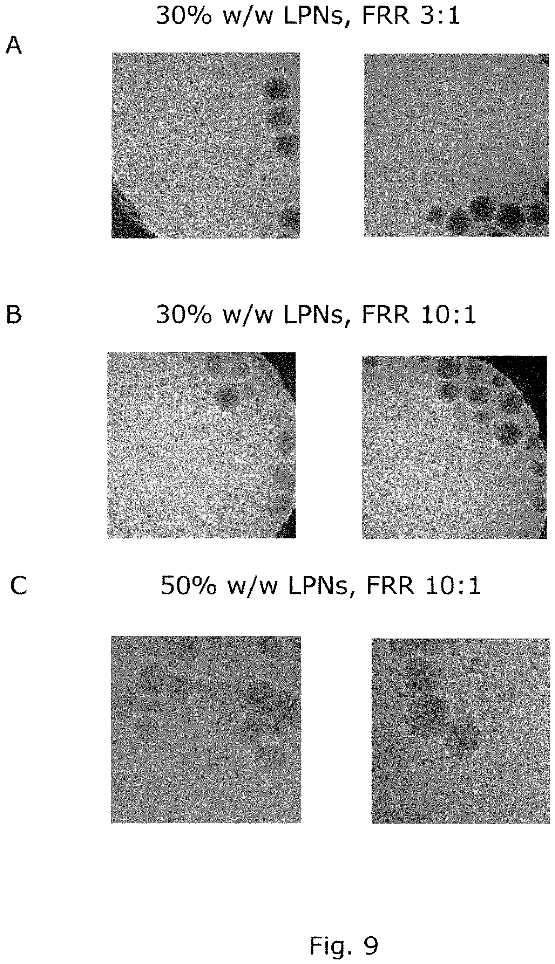

**Figure 9A** shows cryoTEM images of 30 % (w/w) LPNs with 7 mol% DMPE-PEG₂₀₀₀ and a *C12-200:FLuc* mRNA weight ratio of 10:1 prepared using a FRR of 3:1.
**Figure 9B** shows cryoTEM images of 30 % (w/w) LPNs with 7 mol% DMPE-PEG₂₀₀₀ and a *C12-200:FLuc* mRNA weight ratio of 10:1 prepared using a FRR of 10:1.
**Figure 9C** shows cryo-TEM images of 50% w/w LPNs with 7 mol% DMPE-PEG₂₀₀₀ and a C12-200:*FLuc* mRNA weight ratio of 10:1 prepared using a FRR of 10:1

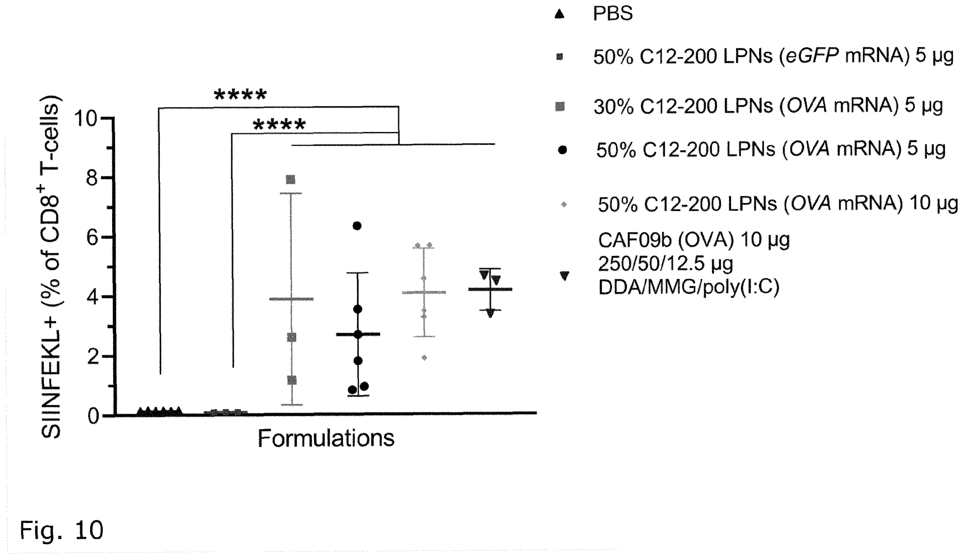

**Figure 10** shows the percentage of OVA₂₅₇₋₂₆₄-specific MHC-I Tet⁺CD8⁺CD44⁺ T cells following the indicated treatments. The order of the treatments from left to right in the plot diagram is PBS, 50% C12-200 LPNs (eGFP mRNA) 5 µg, 30% C12-200 LPNs (*OVA* mRNA) 5 µg, 50% C12-200 LPNs (*OVA* mRNA) 5 µg, 50% C12-200 LPNs (*OVA* mRNA) 10 µg, and CAF09b (OVA protein) 5 µg [250/50/12.5 µg DDA/MMG/poly(I:C)]. Mean values ± SD, n = 6. One-way ANOVA with Tukey's Multiple Comparison test. ^{∗∗∗∗}*p* < 0.0001.

The present invention will now be described in more detail in the following.

### Detailed description of the invention

### Definitions

Prior to discussing the present invention in further details, the following terms and conventions will first be defined:

### Nanoparticle

A nanoparticle is a spherical, polymeric particle composed of natural or synthetic polymers. The nanoparticle may also comprise lipids or lipid-like materials. A nanoparticle ranges in size between 10 and 500 nm.

### Lipid-polymer hybrid nanoparticle

In the present context, the term "lipid-polymer hybrid nanoparticle", abbreviated LPN, relates to a nanoparticle comprising both lipids and a polymer.

### Cationic or cationically ionisable lipid or lipid-like material

In the present context, the term "cationic or cationically ionisable lipid or lipid-like material" refer to lipids or lipid-like materials that are either permanently positively charged (cationic) or becomes positively charged whenever the pH changes (cationically ionisable). An "ionisable lipid or lipid-like material" is a lipid that is positively charged at acidic pH and neutral at physiological pH (∼7.4). Ionisable lipids or lipid-like materials are positively charged at acidic pH, where they are used to condense and load RNAs into nanoparticles, but are neutral at physiological pH to minimise toxicity. They are protonated in the acidic endosomes after cellular uptake, and interact with anionic endosomal phospholipids to form cone-shaped ion pairs that are not compatible with a lipid bilayer. These cationic-anionic lipid pairs drive the transition from a bilayer structure to an inverted hexagonal H_{II} phase, which is suggested to facilitate membrane fusion/disruption, endosomal escape, and cargo release into the cytosol.

The terms "lipid" and "lipid-like material" are broadly defined herein as molecules, which comprise one or more hydrophobic moieties or groups and optionally also one or more hydrophilic moieties or groups. Molecules comprising hydrophobic moieties and hydrophilic moieties are also frequently denoted as amphiphiles. Lipids are usually poorly soluble in water. In an aqueous environment, the amphiphilic nature allows the molecules to self-assemble into organised structures and different phases. One of those phases consists of lipid bilayers, as they are present in vesicles, unilamellar/multilamellar liposomes, or membranes in an aqueous environment. Hydrophobicity can be conferred by the inclusion of apolar groups that include, but are not limited to, long-chain unsaturated and saturated aliphatic hydrocarbon groups and such groups substituted by one or more aromatic, cycloaliphatic, or heterocyclic group(s). The hydrophilic groups may comprise polar and/or charged groups and include carbohydrates, phosphate, carboxylic, sulphate, amino, sulfhydryl, nitro, hydroxyl, and other like groups.

### Lipid-like material or lipid-like conjugate

The term "lipid-like material", "lipid-like compound", "lipid-like conjugate" or "lipid-like molecule" relates to substances that structurally and/or functionally relate to lipids, but may not be considered as lipids in a strict sense. For example, the term includes compounds that are able to form amphiphilic layers as they are present in vesicles, unilamellar/multilamellar liposomes, or membranes in an aqueous environment, and includes surfactants, or synthesised compounds with both hydrophilic and hydrophobic moieties. Generally speaking, the term refers to molecules, which comprise hydrophilic and hydrophobic moieties with different structural organisation, which may or may not be similar to that of lipids. As used herein, the term "lipid" is to be construed to cover both lipids and lipid-like materials, unless otherwise indicated herein, or clearly contradicted by context.

### Helper lipid

Helper lipids are a class of lipid molecules that increase particle stability and modulates the fluidity of nanoparticles. Several classes of molecules can be used as helper lipids, *e.g.,* phospholipids, as exemplified by 1,2-distearoyl-*sn*-glycero-3-phosphocholine (DSPC), and DOPE, and sterols, exemplified by cholesterol.

### Lipopolymer

In the present context, the term "lipopolymer" is any polymer covalently linked to a lipid (fatty acid or steroid) moiety, *e.g*., PEGylated lipids or polysarcosine lipids.

### Monomycoloyl glycerol (MMG) and MMG analogue

The mycobacterial cell wall lipid monomycoloyl glycerol (MMG) or an analogue thereof is a glycerolipid.

Glycerolipids like MMG possess immunopotentiating properties and can enhance immune responses. MMG is too toxic for human use. Therefore, well-tolerated synthetic analogues of MMG have been developed.

The synthetic analogue, referred to as MMG-1, consists of a hydrophilic glycerol headgroup and a lipid acid, displaying two hydrophobic saturated C14/C15 alkyl tails, linked via an ester bond. Furthermore, an array of MMG analogues, differing in the alkyl chain lengths (MMG-2; C16/C17, MMG-3; C10/C11, and MMG-4; C6/C7), or with respect to the stereochemistry of the headgroup (MMG-5; 2S) and the lipid tail (MMG-6, MMG-7), has been designed.

MMG is preferably the synthetically manufactured glycerolipid, MMG-1. Monomycoloyl glycerol-1 (MMG-1) is a shorter synthetic analogue of the mycobacterial cell wall lipid, monomycoloyl glycerol (MMG). MMG-1 binds to the pattern-recognition receptor (PRR) C-type lectin-type receptor (CLR) called Mincle. Upon incorporation into liposomes, MMG-1 has been shown to display an immunomodulatory effect via activation of dendritic cells (DCs), resulting in increased secretion of the Th1 cytokine interferon gamma (IFN-γ) and the Th17 cytokine interleukin-17 (IL-17). The chemical structure of the preferred MMG analogue is 3-hydroxy-2-tetradecyl-octadecanoic acid-2,3-dihydroxypropyl ester, preferably the (2*R*)-2,3-dihydroxypropyl-3-hydroxy-2-tetradecyloctadecanoate diastereomer. Both MMG-6 and MMG-7 adopt an inverse hexagonal phase (Hu), which might enable higher endosomal escape, and thereby a higher degree of nucleic acid cargo release, than other MMG analogues. Thus, both MMG-6 and MMG-7 are preferred analogues to use in the present invention.

### Cholesterol

Cholesterol is a sterol with the IUPAC name (3*S*,8*S*,9*S*,10*R*,13*R*,14*S*,17*R*)-10,13-dimethyl-17-[(2*R*)-6-methylheptan-2-yl]-2,3,4,7,8,9,11,12,14,15,16,17-dodecahydro-1*H*-cyclopenta[a]phenanthren-3-ol. Cholesterol is biosynthesised by all animal cells and constitutes an essential structural component of animal cell membranes by providing stability. Cholesterol is often included in lipid nanoparticle (LNP) compositions. In LNPs, cholesterol plays several roles including filling gaps in the particles, limiting LNP-protein interactions, maintaining membrane integrity, and possibly promoting membrane fusion.

### Nucleic acid

In the present context, the term "nucleic acid" is a deoxyribonucleic acid (DNA) or preferably a ribonucleic acid (RNA), more preferably mRNA. Nucleic acids may also be referred to as a "nucleic acid cargo", since they do not form a part of the LPN carrier but instead are loaded into said LPN. Nucleic acids include, but are not limited to, genomic DNA, plasmid DNA, cDNA, mRNA, and recombinantly produced or chemically synthesised molecules. A nucleic acid according to the invention may be in the form of a molecule, which is single stranded or double stranded, and linear or closed covalently to form a circle. A nucleic acid can be employed for introduction into cells, i.e. transfection of cells, for example, in the form of mRNA, which can be prepared enzymatically by *in vitro* transcription from a DNA template. The RNA can, moreover, be modified before application by stabilising sequences, capping, and/or polyadenylation.

### RNA molecule

In the context of the present invention, the term "RNA molecule" relates to a molecule, which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double-stranded RNA, single-stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as to the end(s) of an RNA or internally, for example at one or more nucleotides of the RNA. Nucleotides in RNA molecules can also comprise non-standard nucleotides, *e.g*., non-naturally occurring nucleotides or chemically synthesised nucleotides or deoxynucleotides.

### Organic phase

The term "organic phase" refers to an organic solvent in which apolar solutes are solubilised. In the present context, the organic solvent comprising acetonitrile supplemented with acetic acid is used to dissolve lipids, such as a cationic or cationically ionisable lipid or lipid-like material, a helper lipid, a lipopolymer, and the one or more variants of PLGA.

### Aqueous phase

The term "aqueous phase" refers to an aqueous solvent in which polar solutes are solubilised. In the present context, the term relates to an aqueous phase obtained by diluting at least one nucleic acid in an aqueous solvent, which is a buffer, preferably at a pH of 3.0-7.8.

### Buffer

A buffer (more precisely, pH buffer or hydrogen ion buffer) is an acid or a base aqueous solution consisting of a mixture of a weak acid and its conjugate base, or vice versa. The pH of a buffer changes very little when a small amount of strong acid or base is added to it. Buffer solutions are used as a means of maintaining pH at a nearly constant value in a wide variety of chemical applications. In a preferred embodiment, the buffer is a citrate buffer. A citrate buffer is a buffered mixture of sodium citrate and citric acid. Citrate buffers are commonly used for RNA isolation, due to their ability to prevent base hydrolysis and prevent Mg²⁺ dependent-RNA degradation by chelating Mg²⁺. In a preferred embodiment of the present invention, the pH of the citrate buffer is in the range of 3.0-4.0.

### Nanoprecipitation

Nanoprecipitation is the process, which is used to generate nanoparticles by mixing an organic phase comprising different apolar solutes with an aqueous solvent phase comprising at least one nucleic acid. Nanoprecipitation can be performed by, but is not limited to, mixing the two phases using pipette mixing, a T-mixer, microfluidic mixing, or impingement jet mixers.

### Microfluidic mixing

Microfluidic mixing can be used to achieve a thorough and rapid mixing of multiple solvents in microscale devices. In the present invention, microfluidic mixing is used to generate nanoparticles by mixing an organic phase comprising different apolar solutes with an aqueous phase comprising at least one nucleic acid. Said phases are separately injected into a microfluidic chip containing small channels with sizes of ten to hundreds of micrometers.

### Flow rate ratio

The FRR ratio (FRR) is a dimensionless number that represents the ratio between the volumes of the aqueous phase and the organic phase, which flow through the microfluidic channel per unit time. The term "flow rate ratio" or "FRR" is in the present context used in regards to microfluidic mixing, wherein the FRR is the ratio between the flow rate of the aqueous phase and the flow rate of the organic phase. Hence, a FRR of 3:1 means the aqueous phase flows through the microfluidic mixing chip three times faster than the organic phase. The FRR has been shown to affect the resulting particle size and internal structure of the nanoparticles, which might affect mRNA expression.

### Total flow rate

In the present context, the term "total flow rate" is used in relation to microfluidic mixing, wherein said term denotes the total flow rate of the aqueous phase and the organic phase in the microfluidic mixing chip.

### Antigen

In the present context, the term "antigen" refers to a molecule, such as an immunogenic peptide, that can induce an immune response. The immune response generated by the antigen may be B-cell driven (antibody-mediated immune response) and/or T-cell driven (cellular immune response).

### Administration

The term "administration" in the context of the present invention means administration in various modes either by systemic administration, such as intramuscular, subcutaneous, intradermal, or intraperitoneal injection, or in delivery formulation or devices for e.g., topical-, intradermal-, intranasal-, sublingual-, oral- or pulmonary administration.

The nanoparticle and/or vaccine composition according to the present invention is typically administered by parenteral administration, more typically by subcutaneous or intramuscular injection in the range of once per two weeks, to once or twice per month, to once or twice per year.

### A nanopartide composition

An object of the present invention relates to a nanoparticle composition with a high colloidal stability, which displays an efficient intracellular delivery of nucleic acids. Thus, an aspect of the invention relates to a nanoparticle composition comprising a cationic or cationically ionisable lipid or lipid-like material, a helper lipid, a lipopolymer, and one or more variants of poly(D,L-lactic-*co*-glycolic acid) (PLGA),
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9‴′,9‴ʺ- ((((benzene-1,3,5-tricarbonyl)yris(azanediyl)) tris (propane-3,1-diyl)) tris(azanetriyl))hexanonanoate (FTT5), 1,2-dioleoyl-3-trimethylamonniumpropane (DOTAP), dimethyldioctadecylammonium bromide (DDAB), 1,2-dioleoyl-3-dimethylammonium-chloride (DODAC), and 1,2-di-O-octadecenyl-3-trimethylammonium propane (DOTMA), or any mixture thereof,
wherein the helper lipid is selected from the group consisting of 1,2-dioleoyl-*sn-*glycero-3-phosphoethanolamine (DOPE), 1,2-dioleoyl-*sn*-glycero-3-phosphocholine (DOPC), 1,2-dipalmitoyl-*sn*-glycero-3-phosphocholine (DPPC), 1,2-distearoyl-*sn*-glycero-3-phosphocholine (DSPC), and 1,2-ditetradecanoyl-*sn-*glycero-3-phosphocholine (DMPC), or any mixture thereof, and
wherein the lipopolymer is a polyethylene glycol (PEG)- or polysarcosine-lipid conjugate, or a PEG- or polysarcosine-lipid like conjugate, or any mixture thereof.

An alternative aspect of the present invention relates to a nanoparticle composition consisting of a cationic or cationically ionisable lipid or lipid-like material, a helper lipid, a lipopolymer, and one or more variants of poly(D,L-lactic-*co*-glycolic acid) (PLGA),
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9‴′,9‴ʺ- ((((benzene-1,3,5-tricarbonyl)yris(azanediyl)) tris (propane-3,1-diyl)) tris(azanetriyl))hexanonanoate (FTT5), 1,2-dioleoyl-3-trimethylamonniumpropane (DOTAP), dimethyldioctadecylammonium bromide (DDAB), 1,2-dioleoyl-3-dimethylammonium-chloride (DODAC), and 1,2-di-O-octadecenyl-3-trimethylammonium propane (DOTMA), or any mixture thereof,
wherein the helper lipid is selected from the group consisting of 1,2-dioleoyl-*sn-*glycero-3-phosphoethanolamine (DOPE), 1,2-dioleoyl-*sn*-glycero-3-phosphocholine (DOPC), 1,2-dipalmitoyl-*sn*-glycero-3-phosphocholine (DPPC), 1,2-distearoyl-*sn*-glycero-3-phosphocholine (DSPC), and 1,2-ditetradecanoyl-*sn-*glycero-3-phosphocholine (DMPC), or any mixture thereof, and
wherein the lipopolymer is a polyethylene glycol (PEG)- or polysarcosine-lipid conjugate, or a PEG- or polysarcosine-lipid like conjugate, or any mixture thereof.

The lipopolymer of the nanoparticle compositions contributes to colloidal stabilisation of said nanoparticles. Often polyethylene glycol (PEG)-lipid conjugates or PEG-lipid like conjugates are used as the lipopolymer in nanoparticles, particularly in LPN or LNP compositions. Thus, in an embodiment, the lipopolymer is a polyethylene glycol (PEG)-lipid conjugate or a PEG-lipid like conjugate.

However, polysarcosine-lipid or polysarcosine-lipid like conjugates are being considered as a promising alternative to PEG-lipid or PEG-lipid like conjugates, since polysarcosine combines PEG-like properties, *e.g.,* excellent solubility in water, protein resistance, low cellular toxicity, and a non-immunogenic character, while being based on endogenous material. In addition, some people develop allergies to PEG, in which case it is particularly relevant to have an alternative to PEG-lipid or PEG-lipid like conjugates. Polysarcosine-functionalised LNPs have been reported in the literature, and polysarcosine-based LNPs enable safe and efficient delivery of mRNA, thus signifying an excellent basis for the development of PEG-free RNA therapeutics. Hence, another embodiment relates to the nanoparticle composition, wherein the lipopolymer is selected from the group consisting of 1,2-dimyristoyl-*sn*-glycero-3-phosphoethanolamine-N-[methoxy(polyethylene glycol)-2000] (DMPE-PEG₂₀₀₀), 1,2-dimyristoyl-*rac-*glycero-3-methoxypolyethylene glycol-2000 (DMG-PEG₂₀₀₀), 1,2-distearoyl-*sn-*glycero-3-phosphoethanolamine-N-[amino(polyethylene glycol)-2000] DSPE-PEG₂₀₀₀, 2-[(polyethylene glycol)-2000]-N,N-ditetradecylacetamide (ALC-0159), N-(methylpolyoxyethylene oxycarbonyl)-1,2-dipalmitoyl-*sn*-glycero-3-phosphoethanolamine (DPPE-PEG), 1,2-distearoyl-*rac*-glycerol-3-methoxypolyethylene glycol (DSG-PEG), N-tetradecyl polysarcosine25, N-hexadecyl polysarcosine25, N-octadecyl polysarcosine25, N-dodecyl polysarcosine25, N,N-ditetradecylamine-N-succinyl[methyl(polysarcosine)45], N,N-ditetradecylamine-N-succinyl[methyl(polysarcosine)35], and N,N-ditetradecyl-polysarcosine-25, or any mixture thereof.

In an embodiment, the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), tetrakis(8-methylnonyl) 3,3',3",3‴-(((methylazanediyl)bis(propane-3,1-diyl))bis(azanetriyl))tetrapropionate (306Oi10), 1,2-dioleoyl-3-trimethylamonniumpropane (DOTAP), dimethyldioctadecylammonium bromide (DDAB), 1,2-dioleoyl-3-dimethylammonium-chloride (DODAC), or 1,2-di-O-octadecenyl-3-trimethylammonium propane (DOTMA), preferably C12-200.

In another embodiment, the helper lipid is 1,2-dioleoyl-*sn*-glycero-3-phosphocholine (DOPC), or 1,2-dioleoyl-*sn*-glycero-3-phosphoethanolamine (DOPE), preferably DOPE.

In yet another embodiment, the lipopolymer is 1,2-dimyristoyl-*sn*-glycero-3-phosphoethanolamine-N-[methoxy(polyethylene glycol)-2000] (DMPE-PEG₂₀₀₀), N-(methyl polyoxyethylene oxycarbonyl)-1,2-dipalmitoyl-*sn*-glycero-3-phosphoethanolamine (DPPE-PEG), 1,2-distearoyl-*rac*-glycerol-3-methoxypolyethylene glycol (DSG-PEG), N-tetradecyl polysarcosine25, 1,2-distearoyl-*sn*-glycero-3-phosphoethanolamine-N-[amino(polyethylene glycol)-2000] (DSPE-PEG₂₀₀₀), or 1,2-distearoyl-sn-glycero-3-phosphoethanolamine-N-[amino(polyethylene glycol)-2000] (DMG-PEG₂₀₀₀), preferably DMPE-PEG₂₀₀₀ or DMG-PEG₂₀₀₀.

In a preferred embodiment, the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), the helper lipid is 1,2-dioleoyl-*sn*-glycero-3-phosphoethanolamine (DOPE), and the lipopolymer is 1,2-dimyristoyl-*sn*-glycero-3-phosphoethanolamine-N-[methoxy(polyethylene glycol)-2000] (DMPE-PEG₂₀₀₀).

### Defining the variants of poly(D,L-lactic-co-glycolic acid) (PLGA)

The inventors tested different types of PLGA in Example 7. Said variants had different lactic acid:glycolic acid molar ratios. In general, PLGA types with a higher glycolide content are more hydrophilic and they degrade faster, whereas PLGA types with a higher lactide content degrade very slowly. Since the transfection efficiency is dependent on the degradation rate of PLGA, the PLGA variants with a high lactide content are preferred over PLGA variant with a high glycolide content. Thus, in an embodiment, the one or more variants of poly(D,L-lactic-co-glycolic acid) (PLGA) have a lactic acid:glycolic acid molar ratio in the range of 45:55 to 90:10, such as 50:50, or such as 75:25, preferably 50:50. Furthermore, as is evident from table 1, the average molecular weight of the tested variants of PLGA were also rather different. Hence, in an embodiment, the one or more variants of poly(D,L-lactic-co-glycolic acid) (PLGA) have an average molecular weight in the range of 1 kDa to 100 kDa, such as 2 kDa to 50 kDa, such as 5 kDa to 25 kDa, preferably 24 kDa to 38 kDa.

The inventors have successfully generated LPN compositions comprising PEG-PLGA co-polymers (data not shown). Thus, in another embodiment, the one or more variants of poly(D,L-lactic-co-glycolic acid) (PLGA) is a co-polymer, such as a PEG-PLGA co-polymer.

In an embodiment, the one or more variants of poly(D,L-lactic-co-glycolic acid) (PLGA) have at least one ester-end group modification, or at least one acid-end group modification. Different PLGA types have been assessed for LPN-mediated mRNA delivery by Zhao *et al.,* who used the lipid TT3, and the transfection efficiency of mRNA increased when more hydrophobic PLGA types were used. This could be related to the results obtained in example 7. As the ester-terminated PLGA is more hydrophobic, it should essentially give a higher response, which is evident from **P1** and **P3.** They both mediate higher luciferase expression than **P2** and **P4,** respectively. Also, acid-terminated PLGA is more prone to hydrolysis, which could also lead to a reduced stability and hence, lower response. As shown in example 7, LPNs formulated with P1 (50:50, 24-38 kDa, ester terminated) mediated a 2 to 3-fold higher expression than the other PLGA types (Figure 6B). In a preferred embodiment, the one or more variants of poly(D,L-lactic-*co*-glycolic acid) (PLGA) have at least one ester-end group modification. In an embodiment, the content of one or more variants of poly(D,L-lactic-co-glycolic acid) (PLGA) is in the range of 15-80% (w/w), preferably 30-70% (w/w) relative to the total lipid or lipid-like molar content in the nanoparticle composition.

### Defining the nanoparticle composition - Cholesterol and monomycoloyl glycerol (MMG)

The nanoparticles, in particular the LPNs, generated in the Examples did not comprise cholesterol. Cholesterol is one of the four components that LNPs usually comprise, and since the present invention aims at producing LPNs, adding cholesterol to the nanoparticles may result in phase separation and the formation of LNPs, which are not more colloidally stable than standard LNPs. Thus, in an embodiment, the nanoparticle composition does not comprise cholesterol.

The LPNs of the present invention could comprise an immunopotentiating material, e.g., a ligand for a pattern-recognition receptor (PRR). Thus, in an embodiment the nanoparticle composition further comprises a monomycoloyl glycerol (MMG) analogue. In an embodiment, the monomycoloyl glycerol (MMG) analogue is selected from the group consisting of MMG-1, MMG-2, MMG-3, MMG-4, MMG-5, MMG-6, and MMG-7, or any mixture thereof. In a preferred embodiment, the monomycoloyl glycerol (MMG) analogue is MMG-1, MMG-6, and/or MMG-7, preferably MMG-1. In another embodiment, the monomycoloyl glycerol (MMG) analogue is added to a molar content in the range of 5 mol% to 70 mol%, such as 8 mol% to 60 mol%, such as 10 mol% to 50 mol%, relative to the total molar content of the nanoparticle composition.

### Defining the content of the cationic or cationically ionisable lipid or lipid-like material, the helper lipid and the lipopolymer

The content of the cationic or cationically ionisable lipid or lipid-like material, the helper lipid, and the lipopolymer may vary greatly based on what type of lipids or lipid-like material or lipopolymer is used. Thus, in an embodiment, the cationic or cationically ionisable lipid or lipid-like material is added to a molar content in the range of 50 mol% to 90 mol%, such as 55 mol% to 85 mol%, preferably 60 mol% to 80 mol%, more preferably 65 mol% to 75 mol% relative to the total lipid or lipid-like molar content in the nanoparticle composition. In another embodiment of the present invention, the helper lipid is added to a molar content in the range of 5.0 mol% to 40 mol%, such as 10 mol% to 35 mol%, preferably 20 mol% to 30 mol%, relative to the total lipid or lipid-like molar content in the nanoparticle composition. In a further embodiment, the lipopolymer is added to a molar content in the range of 0.5 mol% to 50 mol%, such as 0.8 mol% to 40 mol%, such as 1 mol% to 30 mol%, such as 2 mol% to 20 mol%, such as 5 mol% to 10 mol%, preferably 3 mol% to 8 mol%, more preferably 2 mol% to 7 mol% relative to the total lipid or lipid-like molar content in the nanoparticle composition.

### Defining the nanoparticle composition - Nucleic acid

The nanoparticle composition of the present invention could be used as a delivery system for several cargos, such as peptides or nucleic acids. Thus, in an embodiment, the nanoparticle composition further comprises at least one nucleic acid. The nucleic acids may be a mixture of different nucleic acids, or it may be the same nucleic acid. Hence, in an embodiment, the at least one nucleic acid is a mixture of nucleic acids. Depending on the therapeutic purpose, several different nucleic acids may be relevant cargoes, such as mRNAs for use in an mRNA vaccine or siRNAs for use to knock down protein(s) of interest, or gRNAs for use in the CRISPR/Cas9 technology to knock out protein(s) of interest. Hence, in an embodiment of the present invention, the at least one nucleic acid is selected from the group consisting of messenger RNA (mRNA), self-amplifying RNA, circular RNA (circRNA), plasmid DNA (pDNA), small interfering RNA (siRNA), single guide RNA (sgRNA), guide RNA (gRNA), long non-coding RNA (IncRNA), small activating RNA (saRNA), and splice-switching antisense oligonucleotide (ASO).

An siRNA could for instance be used to knock down the expression of tumour necrosis factor-a (TNF-o). Thus in an embodiment, the at least one nucleic acid is an siRNA, preferably targeting tumour necrosis factor-a (TNF-a) (SEQ ID NO.: 3, SEQ ID NO.: 4). In a preferred embodiment, the at least one nucleic acid is an RNA molecule or a mixture of RNAs. The nucleic acids used in the examples are RNAs, specifically mRNAs. Thus, in a more preferred embodiment, the at least one nucleic acid is an mRNA or a mixture of mRNAs.

The nucleic acids used as cargoes in these LNPs may be modified, either by having chemically modified nucleosides or by having different bonds. These modifications can for example help stabilise the nucleic acids or make them more resistant to nucleases. Thus, in an embodiment, the at least one nucleic acid may comprise at least one chemically modified nucleoside, such as a pseudouridine, an N1-methyl pseudouridine, and a nucleoside with a 2'-O-methylation. In a further embodiment, the at least one nucleic acid may comprise phosphodiester bonds, phosphorothioate bonds, or a mixture thereof, preferably phosphodiester bonds.

### Vaccine composition

The nanoparticle composition of the present invention is particularly useful as a vaccine composition, since the nanoparticle composition is colloidally stable and displays efficient intracellular delivery of nucleic acids. Thus, an aspect of the present invention relates to a vaccine composition comprising the nanoparticle composition according to any one of the preceding claims and at least one nucleic acid encoding an antigen. In an embodiment, the antigen is an antigen from a pathogen causing an infectious disease. In a further embodiment, the antigen is selected from the group consisting of corona virus antigens, such as SARS-CoV and MERS-CoV antigens, such as SARS-CoV2 spike protein (SEQ ID NO.: 5) or receptor binding domain (RBD), *Mycobacterium tuberculosis* antigens (SEQ ID NO.: 8-9), *Plasmodium falciparum* antigens (SEQ ID NO.: 10), respiratory syncytial virus (RSV) antigens (SEQ ID NO.: 11), Ebolavirus antigens, Marburg virus antigens, Lassa virus antigens, Nipah virus antigens, Zika virus antigens, Crimean-Congo haemorrhagic fever orthonairovirus antigens, human papilloma virus (HPV) antigens, and influenza antigens. As shown in the examples, the nanoparticle composition can effectively deliver an mRNA cargo, and therefore, the LNP composition is particularly useful in an mRNA vaccine. Hence, in an embodiment, the antigen is encoded by an mRNA.

### Use of the vaccine composition

An aspect relates to the vaccine composition according to the present invention for use in the prevention and/or treatment of an infectious disease. In an embodiment, the infectious disease is selected from the group consisting of tuberculosis, COVID-19, MERS-CoV infection, SARS-CoV infection, malaria, RSV infection, Ebola, Marburg virus infection, Lassa fever, Nipah virus infection, Zika virus infection, Crimean-Congo haemorrhagic fever, HPV infection, and influenza.

### Vaccine administration

The nanoparticle composition was administered to mice using subcutaneous injection in example 9 and 11. However, as a vaccine composition said nanoparticle composition according to the present invention and at least one nucleic acid encoding an antigen could be administered using other routes of administration. Thus, in an embodiment, the vaccine is administered to a subject by intradermal, intraperitoneal, intravenous, intramuscular, or subcutaneous injection. Another embodiment relates to the vaccine composition for use according to the present invention, wherein the subject is a mammal, such as a human, a non-human primate, a monkey, a calf, a pig, a horse, a sheep, a goat, a mink, a ferret, a hamster, a cat, a bird, or a dog. In a preferred embodiment, the subject is a human.

In an embodiment, the vaccine is administered as a single dose. However, vaccines are often given in a multidose regimen, *e.g.,* the COVID-19 mRNA vaccines Spikevax^{®} and Comirnaty^{®}. Thus, in an embodiment, the vaccine is administered as at least two doses, such as at least three doses.

### Process for obtaining the nanoparticle composition

As demonstrated in example 2, the process for preparing LPNs comprising one or more variants of PLGA can be optimised by dissolving the cationic or cationically ionisable lipid or lipid-like material, the helper lipid, the lipopolymer, and the one or more variants of PLGA in acetonitrile supplemented with acetic acid. Thus, an aspect relates to a process for obtaining the nanoparticle composition according to the present invention, said process comprising the steps of:
a) Providing an cationic or cationically ionisable lipid or lipid-like material, a helper lipid, a lipopolymer, one or more variants of poly(D,L-lactic-*co-*glycolic acid) (PLGA), and at least one nucleic acid;
b) Dissolving the cationic or cationically ionisable lipid or lipid-like material, the helper lipid, the lipopolymer, and the one or more variants of PLGA of step a) in an organic solvent comprising acetonitrile supplemented with acetic acid, thereby providing an organic phase;
c) Diluting the at least one nucleic acid of step a) in an aqueous solvent comprising a buffer with a pH within the range of 3.0 to 7.8, thereby providing an aqueous phase;
d) Mixing the organic phase of step b) with the aqueous phase of step c) to obtain nanoparticles by nanoprecipitation;
e) Performing filtration, preferably tangential flow filtration or dialysis, of the nanoparticles of step d) to obtain a nanoparticle composition;

wherein the cationic or cationically ionisable lipid or lipid-like material is selected from the group consisting of 1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), dilinoleylmethyl-4-dimethylaminobutyrate (DLin-MC3-DMA), N1,N3,N5-tris(3-(didodecylamino)propyl)benzene-1,3,5-tricarboxamide (TT3), 2,2-dilinoleyl-4-dimethylaminoethyl-[1,3]-dioxolane (DLin-KC2-DMA), hexa(octan-3-yl) 9,9',9",9‴,9‴′,9‴ʺ- ((((benzene-1,3,5-tricarbonyl)yris(azanediyl)) tris (propane-3,1-diyl)) tris(azanetriyl))hexanonanoate (FTT5), and 1,2-dioleoyl-3-trimethylamonniumpropane (DOTAP), or any mixture thereof,
wherein the helper lipid is selected from the group consisting of 1,2-dioleoyl-*sn-*glycero-3-phosphoethanolamine (DOPE), DOPC, DOPG, DPPC, DSPC, DPPG, DMPC, MGDG, DGDG, and SQDG, or any mixture thereof, and
wherein the lipopolymer is a PEG- or polysarcosine-lipid conjugate, or a PEG- or polysarcosine-lipid like conjugate, or any mixture thereof.

In an embodiment, said process further comprising the step:
f) Concentrating the nanoparticle composition using a method selected from the group consisting of filtration, centrifugation, vacuum-assisted centrifugation, or any mixture thereof, preferably filtration.

### Defining the cationic or cationically ionisable lipid or lipid-like material, the helper lipid, and the lipopolymer of step a)

An embodiment relates to the process according to the present invention, wherein the lipopolymer is selected from the group consisting of 1,2-dimyristoyl-*sn-*glycero-3-phosphoethanolamine-N-[methoxy(polyethylene glycol)-2000] (DMPE-PEG₂₀₀₀), DMG-PEG₂₀₀₀, DSPE-PEG₂₀₀₀, 2-[(polyethylene glycol)-2000]-N,N-ditetradecylacetamide (ALC-0159), DPPE-PEG, DSG-PEG, ceramide-PEG, DPG-PEG, DOG-PEG, DOPE-PEG, N-tetradecyl polysarcosine25, N-hexadecyl polysarcosine25, N-octadecyl polysarcosine25, N-dodecyl polysarcosine25, N,N-ditetradecylamine-N-succinyl[methyl(polysarcosine)45], N,N-ditetradecylamine-N-succinyl[methyl(polysarcosine)35], and N,N-ditetradecyl-polysarcosine-25, or any mixture thereof. In another embodiment, the cationic or cationically ionisable lipid or lipid-like material is C12-200, the helper lipid is 1,2-dioleoyl-*sn*-glycero-3-phosphoethanolamine (DOPE), and the lipopolymer is 1,2-dimyristoyl-*sn*-glycero-3-phosphoethanolamine-N-[methoxy(polyethylene glycol)-2000] (DMPE-PEG₂₀₀₀).

### Defining the nucleic acid of step a)

An embodiment relates to the process according to the present invention, wherein the at least one nucleic acid is selected from the group consisting of messenger RNA (mRNA), self-amplifying RNA (saRNA), circular RNA (circRNA), plasmid DNA (pDNA), small interfering RNA (siRNA), single guide RNA (sgRNA), guide RNA (gRNA), long non-coding RNA (IncRNA), small activating RNA (saRNA), and splice-switching antisense oligonucleotide (ASO), preferably the at least one nucleic acid is an mRNA.

### Defining the acetonitrile supplemented with acetic acid in step b)

As shown in Example 2 (Figure 1), acetonitrile supplemented with different concentrations of acetic acid was tested as the organic solvent. Thus, in an embodiment, the acetonitrile of step b) is supplemented with acetic acid in a concentration in the range of 1.0% to 10% (v/v), such as 1.5% to 8.0% (v/v), such as 2.0% to 5.0% (v/v), preferably 5.0% (v/v). In another embodiment, the acetonitrile supplemented with acetic acid in step b) is heated to a temperature in the range of 50 °C to 80 °C, such as 55 °C to 75 °C, preferably 60 °C to 70 °C, more preferably 65 °C. In yet another embodiment, the helper lipid is dissolved in ethanol, preferably absolute ethanol with a purity close to 100% before step b).

### Defining the buffer of step c)

An embodiment relates to the process according to the present invention, wherein the pH value of the buffer in step c) is in the range of 3.3 to 6.0, preferably 3.5 to 5.0, preferably the pH is 4.0. The pH value of the buffer might vary depending of what cationic or cationically ionisable lipid or lipid-like material is used. In an embodiment, the buffer is selected from the group consisting of citrate buffer, acetate buffer, Tris buffer, and HEPES buffer.

### Mixing by nanoprecipitation

Nanoprecipitation is a simple method used for encapsulation of both hydrophilic and hydrophobic drugs in nanoparticles. Nanoprecipitation can be performed using several different methods. Thus, in an embodiment, the mixing in step d) is performed using microfluidic mixing, pipette mixing, a T-mixer, or impingement jet mixers. In a preferred embodiment, the mixing in step d) is performed using microfluidic mixing. In another embodiment, the flow rate ratio of the aqueous phase to the organic phase during the microfluidic mixing is in the range of 1:1 to 20: 1, preferably 10:1. In yet another embodiment, the total flow rate during microfluidic mixing is selected from the range of 1.0 mL/min to 20 mL/min, preferably the flow rate is 12 mL/min.

### Defining the filtration of step e)

In an embodiment, the filtration of the nanoparticles in step e) is performed against phosphate-buffered saline or tris buffer, preferably tris buffer at a pH of 7.4.

### Product by process

An aspect relates to a nanoparticle composition obtained using the process of the present invention.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - Materials and methods

### Materials

CleanCap^{®} enhanced green fluorescent protein (*EGFP*) mRNA, firefly luciferase (*FLuc*) mRNA (SEQ ID NO.: 1), and *OVA* mRNA (SEQ ID NO.: 2) (1.0 mg/mL in 1 mM sodium citrate buffer, pH = 6.4) fully substituted with 5-methoxyuridine were purchased from TriLink Biotechnologies (San Diego, CA, USA). Triton^{™} X-100, citric acid, sodium citrate tribasic dihydrate, cholesterol, and 3-(4,5-dimethyl-2-thiazolyl)-2,5-diphenyl-2H-tetrazolium bromide (MTT) were acquired from Sigma Aldrich (St. Louis, MO, USA). Different types of PLGA were purchased from Sigma Aldrich and Wako Pure Chemical Industries (Osaka, Japan) (**Table 1**)**.** Quant-iT^{™} RiboGreen^{®} RNA Reagent, Lipofectamine^{®} MessengerMAX^{™} reagent, Slide-A-Lyzer dialysis cassettes (10,000 MWCO), and Gibco^{™} Dulbecco's phosphate-buffered saline powder (PBS, pH 7.2, 10×) were obtained from Thermo Fisher Scientific (Waltham, MA, USA). The ionisable lipidoid C12-200 was synthesised and purified as reported previously (Love, K.T., *et al.* (2010), and Thanki, K. *et al.* (2017)). The helper lipids 1,2-dioleoyl-sn-glycero-3-phosphoethanolamine (DOPE), 1,2-dimyristoyl-*sn*-glycero-3-phosphoethanolamine-N-[methoxy(PEG)-2000] (DMPE-PEG₂₀₀₀), and 1,2-dimyristoyl-*rac*-glycero-3-methoxyPEG-2000 (DMG-PEG₂₀₀₀) were purchased from Avanti Polar Lipids (Alabaster, AL USA). Cholesterol was obtained from Sigma-Aldrich. HPLC-grade acetonitrile (CH₃CN) and acetic acid (CH₃COOH), and absolute ethanol were obtained from VWR International A/S (Søborg, Denmark). Ultra-pure Type I water (PURELAB Flex, Elga Veolia, UK) filtered with a 0.2 µ filter was used to prepare PBS for dialysis and washing of nanoparticles.

**Table 1: List of PLGA types used to formulate LPNs.**

| PLGA type | Code | Lactide/gl ycolide molar ratio | M_{w} (kDa) | End group modification | Source |
|---|---|---|---|---|---|
| PLGA Resomer^{®} RG 503 | **P1** | 50:50 | 24-38 | Ester | Sigma Aldrich (St. Louis, MO, USA) |
| PLGA Resomer^{®} RG 503H | **P2** | 50:50 | 24-38 | Acid | Sigma Aldrich (St. Louis, MO, USA) |
| PLGA Resomer^{®} RG 502 | **P3** | 50:50 | 7-17 | Ester | Sigma Aldrich (St. Louis, MO, USA) |
| PLGA Resomer^{®} RG 502H | **P4** | 50:50 | 7-17 | Acid | Sigma Aldrich (St. Louis, MO, USA) |
| PLGA 50:50 | **P5** | 50:50 | 20 | Ester | Wako Pure Chemical Industries (Osaka, Japan) |
| PLGA 75:25 | **P6** | 75:25 | 20 | Ester | Wako Pure Chemical Industries (Osaka, Japan) |

### Preparation of mRNA-loaded LPNs and physiochemical characterisation of said LPNs

Lipids and polymer were dissolved in 2% (v/v) CH₃COOH in CH₃CN (except DOPE, which was solubilised in absolute ethanol and then added to CH₃CN), constituting the organic phase, and mRNA was dissolved in either 10 mM or 50 mM sterile citrate buffer in RNase-free water (pH = 3.0 or 4.0), constituting the aqueous phase. The two phases were loaded into syringes and mixed using either a NanoAssemblr^{®} Ignite^{™} or a NanoAssemblr^{®} Benchtop microfluidic mixer (Precision NanoSystems, Vancouver, Canada) to prepare LPNs loaded with mRNA. The resulting LPN formulations were subsequently dialysed using Slide-A-Lyzer dialysis cassettes with a 10,000 MWCO against 1000 volumes of PBS with gentle magnetic stirring at 100 rpm for 4 h at room temperature (RT), followed by 8 h of dialysis against fresh PBS at 4 °C. Five parameters, *i.e.,* (i) the flow rate ratio (FRR), (ii) the content of DMPE-PEG₂₀₀₀, (iii) the C12-200:FLuc mRNA weight ratio, (iv) the total lipid content (lipid:PLGA weight ratio), and (v) the PLGA type **(Table 1),** were varied systematically in one-factor-at-a-time (OFAT) experiments to investigate the effect on physicochemical properties [intensity-weighted mean hydrodynamic diameter (z-average), PDI, and mRNA entrapment efficiency], cell viability, and transfection efficiency. Control LNPs loaded with mRNA were prepared as described previously (Kauffman, KJ *et al.* (2015)). All formulations prepared in the current study are labelled C1-C48 **(Table 2).** Formulations were characterised with respect to z-average and PDI, as reported previously (Thanki, K. *et al.* (2017) and Kauffman KJ. *et al* (2016)). The mRNA entrapment efficiency was quantified using the Quant-iT^{™} RiboGreen^{®} assay, essentially as described previously (Lokras, A. *et al.,* (2021)).

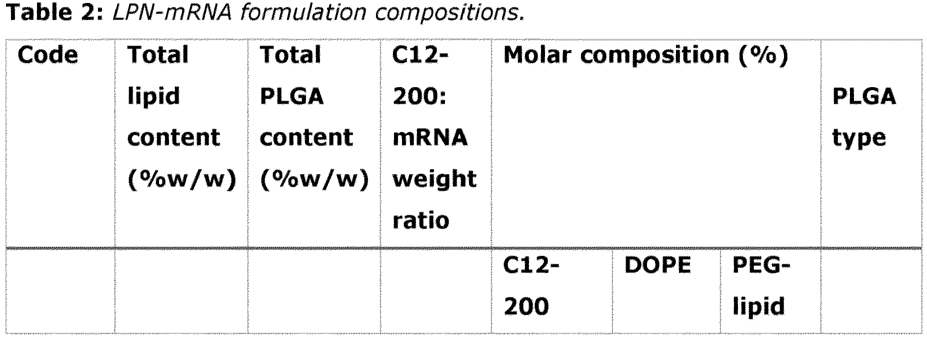

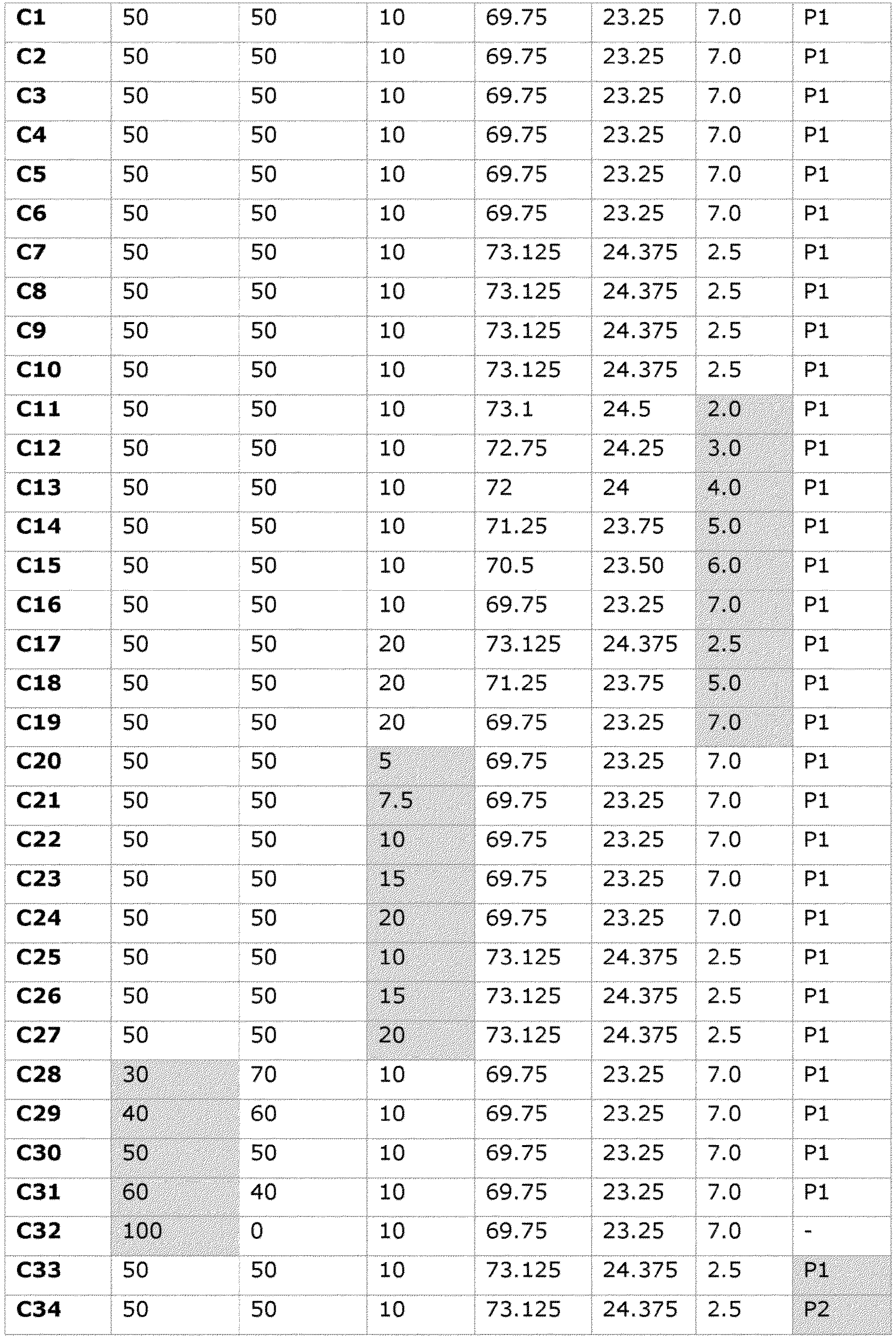

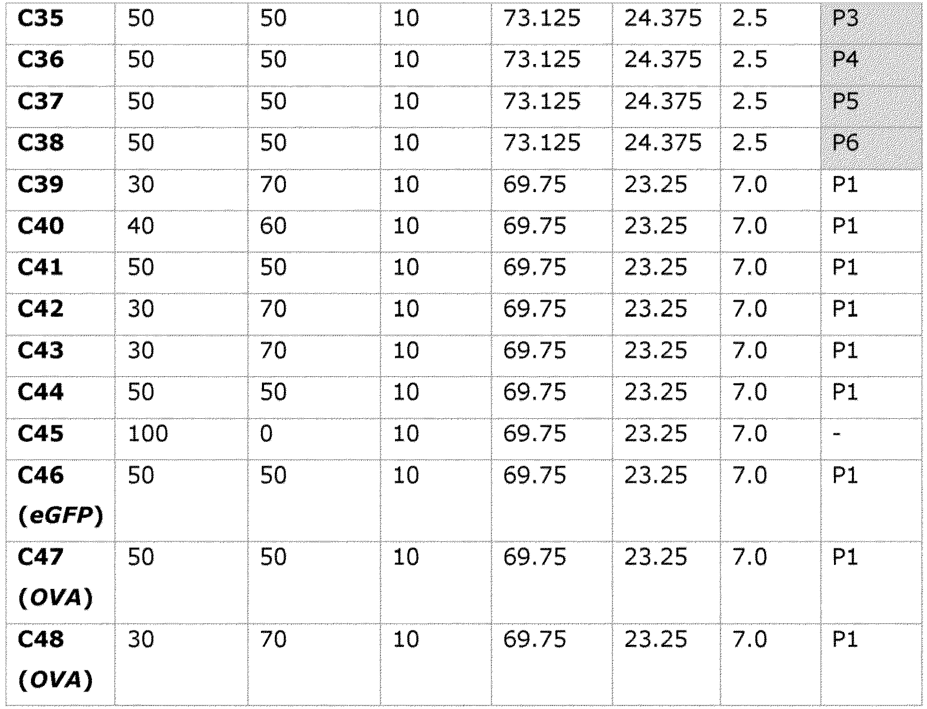

### In vitro luciferase reporter and viability assay

The murine macrophage cell line RAW 264.7 was purchased from the American Type Culture Collection (TIB71, Manassas, VA, USA). The cells were maintained in Dulbecco's Modified Eagle's Medium with high (4.5 g/L) glucose (DMEM+, Fisher Scientific Biotech Line, Slangerup, Denmark) supplemented with 100 U/mL penicillin, 100 µg/mL streptomycin, 2 mM glutamine (all from Sigma-Aldrich), and 10% (v/v) fetal bovine serum (FBS, Gibco, Life Technologies). The cells were grown in a 5% CO₂-95% atmospheric air incubator at 37 °C, the growth medium was renewed every second day, and the cells were subcultured twice a week by detaching them from the culture flask (75 cm², Sigma Aldrich) using a cell scraper. HeLa cells (CRL-11268, American Type Culture Collection) were grown in culture flasks (75 cm², Sigma Aldrich) in the same way as the murine macrophages. RAW 264.7 and HeLa cells were seeded at densities of 4 × 10⁴ and 4 × 10³ cells per well, respectively, in white-walled, clear bottom 96-well plates (Thermo Fisher, Copenhagen, Denmark) in a constant volume of medium of 100 µL. After 24 h, the cells were transfected with LPNs or LNPs loaded with *FLuc* mRNA at mRNA doses ranging from 25 - 200 ng. Expression of *FLuc* was analysed using the ONEGIo^{™} (Promega Biotech AB, Nacka, Sweden) + methylthiazolyldiphenyl-tetrazolium bromide (MTT, Merck Life Sciences, Copenhagen, Denmark) and ONEGIo^{™} + Tox (Promega Corporation, Madison, WI, USA) luciferase reporter and viability assays for RAW 264.7 and HeLa cells, respectively. The cell viability was determined using the MTT assay as reported previously (Lokras, A. *et al.* (2021)). For the ONEGIo^{™} + Tox assay, at 23 h and 30 min, 10 µL 5× cell titer Fluor reagent was added to each well in quadruplicates and incubated at 37 °C for 30 min. The relative fluorescence units (RFUs) were read using a plate reader at an excitation wavelength of 400 nm and an emission wavelength of 505 nm. Live cells show a high fluorescence due to peptide (substrate) cleavage activity, resulting in fluorescence. At 24 h, 25 µL ONEGIo^{™} reagent was added to the wells and incubated at 22 °C for 5 min to lyse the cells. Relative luminescence units (RLU) were measured using a plate reader, and the background from untreated cells was subtracted. The RLU was divided by either percent cell viability or RFU to ensure any changes in luminescence were due to the parameters being investigated, rather than cytotoxicity.

### Statistics

Data was analysed using GraphPad Prism (GraphPad Software version 8, La Jolla, CA, USA). Data are presented as mean values ± standard deviation (SD). Standard curves were analysed by using linear regression for determination of the coefficient of regression (*R*²). Statistically significant differences were assessed by using one-way analysis of variance (ANOVA) or unpaired t-test. The significance of the results is indicated according to p-values (^{∗}*p* < 0.05, ^{∗∗}*p* < 0.01, ^{∗∗∗}*p* < 0.001, and ^{∗∗∗∗}*p* < 0.0001). A p-value ≤ 0.05 was considered statistically significant.

### Example 2 - CH₃CN supplemented with CH₃COOH is required to dissolve a mixture of C12-200 and PLGA

### Aim of study

The aim of this study was to investigate whether the process for preparing the LPNs could be optimised so that both the ionisable lipid C12-200 and PLGA could be dissolved in the same solvent.

### Results

For the design of mRNA-loaded LPNs, the inventors chose C12-200 as model ionisable lipid, because C12-200 has previously been shown to be efficient for small interfering RNA (siRNA) delivery. In addition, the composition of LNPs with C12-200 as ionisable lipid has been optimised for mRNA delivery, and C12-200 is currently considered as a gold standard reference for mRNA research purposes. Furthermore, the inventors chose PLGA as the polymer component because of previous experience with siRNA-loaded LPNs, composed of PLGA and the ionisable lipidoid 98N12-5, which demonstrated promising gene silencing both *in vitro* and *in vivo.* However, in contrast to siRNA-loaded LPNs, initial pilot experiments showed that LPNs composed only of ionisable cationic lipid (98N12-5 or C12-200) and PLGA did not mediate functional cellular delivery of mRNA to the cytosol, neither *in vitro* nor *in vivo* (results not shown). In subsequent pilot experiments, the inventors found that inclusion of two additional phospholipids in the LPNs is essential for mRNA transfection and colloidal stabilisation, *i.e.,* the helper lipid DOPE, and the lipopolymer DMPE-PEG2000.

First, the inventors identified a solvent mixture enabling solubilisation of both C12-200 and PLGA simultaneously. C12-200 is a relatively hydrophobic molecule consisting of five 12-carbon chains, five hydroxyl groups, and a piperazine headgroup. Due to this amphiphilic nature, C12-200 is soluble in ethanol, but is insoluble in CH₃CN, which has a relative polarity of 0.46. In contrast, PLGA is practically insoluble in ethanol, and thus ethanol cannot be used as solvent for the preparation of hybrid nanoparticles using nanoprecipitation. However, PLGA is fully soluble in organic solvents, *e.g.,* acetone and CH₃CN, but acidifying CH₃CN with 2-5% (v/v) CH₃COOH was required for also dissolving C12-200 in CH₃CN **(****Figure 1****).** Upon heating the 2-5% (v/v) CH₃COOH in CH₃CN solution up to 65 °C with sonication, all the components for the LPNs were fully dissolved. Increasing the concentration of glacial acetic acid in acetonitrile from 2 to 5 % (v/v) resulted in approximately 12% increase in *FLuc* mRNA entrapment efficiency.

### Conclusion

The process for preparing LPNs comprising one or more variants of PLGA can be optimised by dissolving the cationic or cationically ionisable lipid or lipid-like material, the lipopolymer, and the one or more variants of PLGA in acetonitrile supplemented with acetic acid. Increasing the concentration of acetic acid from 2% to 5% (v/v) results in an increased mRNA entrapment. The helper lipid is first dissolved in ethanol followed by addition to the said phase above.

### Example 3 - Effect of flow rate ratio (FRR) and microfluidic chip geometry on physicochemical properties and in vitro performance

### Aim of study

The aim of this study was to investigate whether the flow rate ratio (FRR) and the microfluidic chip geometry had an effect on the physicochemical properties and the *in vitro* performance of LPNs.

### Results

The effect of FRR on the physicochemical properties of the mRNA-loaded LPNs and *FLuc* mRNA expression *in vitro* was investigated systematically at a constant C12-*200:FLuc* mRNA weight ratio of 10:1, and a TFR of 12 mL/min. The lipid content of the LPNs was also kept constant at 50% (w/w) total lipids relative to PLGA (**P1**: Resomer^{®} RG 503). The 50% (w/w) total lipids comprised of 70 mol% C12-200, 23 mol% DOPE, and 7 mol% DMPE-PEG2000 for LPNs prepared using the NanoAssemblr^{®} Benchtop^{™} cartridge. For LPNs prepared using the NanoAssemblr^{®} Ignite^{™} cartridge, the same composition was maintained, but with 2.5 mol% DMG-PEG₂₀₀₀. Due to the high costs of reagents, the data presented here are representative of individual formulations prepared once or twice, as indicated, and the formulations were incubated with cells in quadruplicates per mRNA dose. This is common practice in the field, because the microfluidic manufacturing process is highly reproducible.

The z-average of LPNs formulated using the NanoAssemblr^{®} Benchtop^{™} cartridge **(C1-C6)** was reduced from approximately 168 nm to 136 nm when the FRR was increased from 3:1 to 10:1, while the lowest PDI (0.02) was observed at a FRR of 3:1 **(****Figure 2A** and **Table 3).** The FRR did not affect the mRNA entrapment efficiency, which was almost constant at 70%. However, the FRR affected the FLuc expression in HeLa cells at a non-saturating dose of 75 ng mRNA/well **(****Figure 2B****).** In contrast, the z-average of LPNs formulated using the NanoAssemblr^{®} Ignite^{™} cartridge **(C7-C10)** increased from approximately 180 nm to approximately 223 nm when the FRR was increased from 3:1 to 7:1 **(****Figure 2C** and **Table 3).** At a FRR of 10:1, the z-average was approximately 220 nm. Interestingly, the *FLuc* expression in RAW 264.7 murine macrophages at 75 ng and 100 ng mRNA doses was higher for LPNs prepared at a FRR of 10:1 than for LPNs prepared at FRRs of 3:1, 5:1, and 7:1, respectively. Hence, for subsequent experiments, a FRR of 3:1 was chosen for LPN manufacturing using the NanoAssemblr^{®} Benchtop^{™}, while a FRR of 10:1 was chosen for LPN manufacturing using the NanoAssemblr^{®} Ignite^{™}.

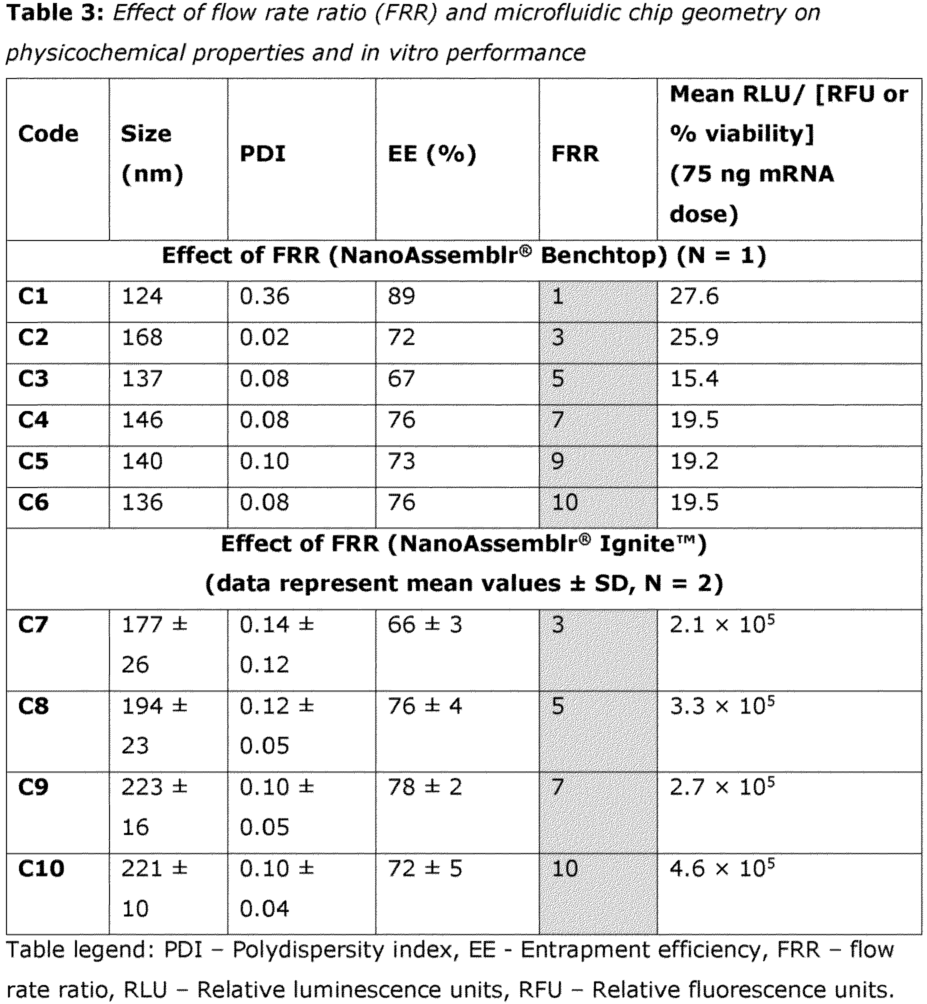

### Conclusion

Both the flow rate ratio and the microfluidic chip geometry has an effect on the physicochemical properties and the *in vitro* performance of the LPNs. For subsequent experiments, a FRR of 3:1 is chosen for LPN manufacturing using the NanoAssemblr^{®} Benchtop^{™}, while a FRR of 10:1 is chosen for LPN manufacturing using the NanoAssemblr^{®} Ignite^{™}.

### Example 4 - Effect of DMPE-PEG₂₀₀₀ and DMG-PEG₂₀₀₀ content on physicochemical properties and in vitro performance

### Aim of study

The aim of this study was to investigate whether different PEGylated lipopolymers could be used in the LPN formulations and the effect of the content of said lipopolymers on the physicochemical properties and the *in vitro* performance.

### Results

Based on previous studies, the inventors hypothesised that addition of PEG-lipid in LPNs would affect the particle characteristics, and *in vitro* and *in vivo* protein production. When the mol% of DMPE-PEG₂₀₀₀ was increased in a step-wise manner from 2 to 7 mol% (**C11-C16, Table 2**), the z-average of the LPNs prepared using the NanoAssemblr^{®} Benchtop^{™} cartridge was reduced from approximately 260 nm to approximately 160 nm, while the PDI was below 0.06 for all tested mol% of DMPE-PEG₂₀₀₀ (**Figure 3A****, Table 4**). The mRNA entrapment efficiency, however, was not affected by the inclusion of DMPE-PEG₂₀₀₀. Increasing the mol% of DMPE-PEG₂₀₀₀ from 2 to 7 mol% in the LPNs caused a linear (*R²* = 0.8535) decrease in the FLuc expression at a dose of 200 ng mRNA (**Figure 3B**)**.** For LPNs prepared using the NanoAssemblr^{®} Ignite^{™}, DMG-PEG₂₀₀₀ was used as the stabiliser (**C17-C19, Table 2**)**.** The z-average of the resulting LPNs was reduced from approximately 270 nm to 130 nm when the DMG-PEG₂₀₀₀ content was increased from 2.5 to 7 mol%. The PDI values for the LPNs prepared using the NanoAssemblr^{®} Ignite^{™} were higher than the PDI values for the LPNs prepared using the NanoAssemblr^{®} Benchtop^{™}, ranging between 0.13 to 0.14 for LPNs, while the mRNA entrapment efficiency was above 70% in all cases (**Figure 3C****, Table 4**)**.** As expected, an increase in the molar content of DMG-PEG₂₀₀₀ from 2.5 mol% to 7.0 mol% resulted in an approximately 4-fold reduction in FLuc expression at an mRNA dose of 75 ng in RAW 264.7 cells **(****Figure 3D****).**

**Table 4: Effect of DMPE-PEG₂₀₀₀ and DMG-PEG₂₀₀₀ content on physicochemical properties and in vitro performance**

| **Code** | **Size (nm)** | **PDI** | **EE (%)** | **FRR** | **Mean RLU/ [RFU or % viability] (75 ng mRNA dose)** |
|---|---|---|---|---|---|
| **Effect of molar percentage of DMPE-PEG2000 (NanoAssemblr^{®} Benchtop) (N = 1)** | | | | | |
| **C11** | 260 | 0.04 | 88 | 3 | 30.6 |
| **C12** | 205 | 0.02 | 85 | 3 | 24.6 |
| **C13** | 216 | 0.05 | 72 | 3 | 20.0 |
| **C14** | 199 | 0.03 | 76 | 3 | 17.8 |
| **C15** | 175 | 0.05 | 74 | 3 | 14.1 |
| **C16** | 160 | 0.05 | 80 | 3 | 11.5 |

| **Effect of molar percentage of DMG-PEG2000 (NanoAssemblr^{®} Ignite^{™}) (data represent mean values ± SD, N** = **2)** | | | | | |
|---|---|---|---|---|---|
| **C17** | 269 ± 17 | 0.13 ± 0.02 | 84 ± 7 | 10 | 3.9 × 10⁵ |
| **C18** | 173 ± 8 | 0.15 ± 0.02 | 73 ± 4 | 10 | 2.1 × 10⁵ |
| **C19** | 130 ± 4 | 0.14 ± 0.03 | 70 ± 5 | 10 | 1.2 × 10⁵ |

| | | | | | |
|---|---|---|---|---|---|
| Table legend: PDI - Polydispersity index, EE - Entrapment efficiency, FRR - flow rate ratio, RLU - Relative luminescence units, RFU - Relative fluorescence units. | | | | | |

### Conclusion

Both DMPE-PEG₂₀₀₀ and DMG-PEG₂₀₀₀ can be used as stabilising lipopolymer in the formulation of PLGA-based LPNs. It is therefore likely that other PEGylated lipids could also be used in the LPN formulations.

### Example 5 - Effect of C12-200:mRNA weight ratio on physicochemical properties and in vitro performance

### Aim of study

The aim of this study was to determine the effect of the C12-200:mRNA weight ratio on selected physicochemical properties and *in vitro* performance of the LPNs.

### Results

Next, the inventors investigated the effect of the C12-200:mRNA weight ratio on the physicochemical properties and *in vitro* performance (**C20-C27, Table 2**). The *z*-average decreased from approximately 284 nm and a PDI of 0.24 for LPNs with a *C12-200:FLuc* mRNA ratio of 5:1 to approximately 166 nm and a PDI of 0.06 for LPNs with a *C12-200:FLuc* mRNA ratio of 20:1 (**Figure 4A** and **C20-C24, Table 5**). As the C12-200:*FLuc* mRNA weight ratio was increased from 5:1 to 20:1, the mRNA entrapment efficiency increased from approximately 47% to 91% **(****Figure 4A****),** which confirms that complexation of mRNA is incomplete at lower *C12-200:FLuc* mRNA weight ratios. The C12-200:mRNA weight ratio also affected *in vitro* FLuc mRNA expression in HeLa cells, with maximum expression at a C12-*200:FLuc* mRNA weight ratio of 10:1 (**Figure 4B**)**.** This highlights that higher entrapment of mRNA in LPNs at the higher C12-200:*FLuc* mRNA weight ratios (15:1 and 20:1) does not necessarily translate to higher mRNA expression *in vitro.*

In contrast, when the C12-200:*FLuc* mRNA weight ratio was increased from 10:1 to 20:1 for LPNs PEGylated with 2.5 mol% DMG-PEG₂₀₀₀ (**C25-C27, Table 2**), the *z*-average increased from approximately 230 nm to 276 nm, while the PDI increased from approximately 0.09 to 0.18 (**Figure 4C**). The *FLuc* mRNA entrapment increased from approximately 68% to 79%, which was expected, because more ionisable lipid is available for complexation with mRNA **(****Figure 4C****).** LPNs formulated at higher C12-200:*FLuc* mRNA weight ratios consistently mediated lower FLuc expression, with the optimal C12-200:*FLuc* mRNA weight ratio being 10:1 (**Figure 4D**). Thus, even though the composition of the LPNs with respect to type of PEG-lipid was different, the optimal C12-200:*FLuc* mRNA weight ratio for *in vitro* FLuc expression for the two different cell lines was found to be 10:1.

**Table 5: Effect of C12-200:mRNA weight ratio on physicochemical properties and in vitro performance**

| **Code** | **Size (nm)** | **PDI** | **EE (%)** | **FRR** | **Mean RLU/RFU (100 ng mRNA dose)** |
|---|---|---|---|---|---|
| **Effect of C12-200:mRNA weight ratio (NanoAssemblr^{®} Benchtop) (N = 1)** | | | | | |
| **C20** | 284 | 0.24 | 47 | 3 | - |
| **C21** | 184 | 0.02 | 63 | 3 | - |
| **C22** | 189 | 0.04 | 77 | 3 | 33.1 |
| **C23** | 179 | 0.05 | 88 | 3 | 20.8 |
| **C24** | 166 | 0.06 | 91 | 3 | 15.5 |

| **Effect of C12-200:mRNA weight ratio ( NanoAssemblr^{®} Ignite^{™}) (N = 1)** | | | | | |
|---|---|---|---|---|---|
| **C25** | 230 ± 13 | 0.10 ± 0.06 | 68 ± 6 | 10 | 6.4 × 10⁵ |
| **C26** | 253 ± 17 | 0.14 ± 0.12 | 75 ± 5 | 10 | 5.5 × 10⁵ |
| **C27** | 276 ± 11 | 0.16 ± 0.46 | 79 ± 4 | 10 | 3.9 × 10⁵ |

| | | | | | |
|---|---|---|---|---|---|
| Table legend: PDI - Polydispersity index, EE - Entrapment efficiency, FRR - flow rate ratio, RLU - Relative luminescence units, RFU - Relative fluorescence units. | | | | | |

### Conclusion

The optimal C12-200:*FLuc* mRNA weight ratio for *in vitro* FLuc expression for the two different cell lines was found to be 10:1 regardless of what PEGylated lipid is used as the lipopolymer.

### Example 6 - Effect of total lipid content on physicochemical properties and in vitro performance

### Aim of study

The aim of this study was to investigate the effect of the total lipid content on selected physicochemical properties and *in vitro* performance of the LPNs.

### Results

Next, the inventors investigated the effect of the total lipid content in LPNs on the z-average, PDI, mRNA entrapment, and FLuc expression in HeLa cells by formulating mRNA-loaded LPNs comprising 30, 40, 50, and 60 % (w/w) of total lipids, relative to PLGA (**C28-C31, Table 2**). The z-average decreased from approximately 225 nm to approximately 181 nm when the total lipid content was increased from 30% to 60% without affecting the PDI, which was below 0.08 for all formulations, except the one without PLGA, wherein the z-average was 87 nm while the PDI was 0.31 (**Figure 5A****, Table 6**). It was expected that the z-average of the LPNs would increase slightly when the lipid content was decreased, because the weight ratio of DMPE-PEG₂₀₀₀ to PLGA would eventually be lower. The mRNA entrapment efficiency was approximately 70% for all formulations but decreased to 26% (**C32, Table 2**) when PLGA was not included in the formulation **(****Figure 5A****, Table 6**). These results suggest that LPNs with a higher content of PLGA (up to 60% w/w) can be formulated. The inventors found no differences in *FLuc* expression mediated by LPNs with lower (30% w/w) or higher (50% w/w) total lipids at doses ranging from 25 - 200 ng mRNA per well (**Figure 5B-C**).

**Table 6: Effect of total lipid content on physicochemical properties and in vitro performance**

| **Code** | **Size (nm)** | **PDI** | **EE (%)** | **FRR** | **Mean RLU/RFU (100 ng mRNA dose)** |
|---|---|---|---|---|---|
| **Effect of total lipid content (% w/w relative to PLGA) (NanoAssemblr^{®} Benchtop) (N = 1)** | | | | | |
| **C28** | 225 | 0.06 | 69 | 3 | 20.8 |
| **C29** | 213 | 0.04 | 71 | 3 | 19.4 |
| **C30** | 189 | 0.04 | 77 | 3 | 21.1 |
| **C31** | 181 | 0.04 | 78 | 3 | 22.3 |
| **C32** | 87 | 0.31 | 27 | 3 | - |

| | | | | | |
|---|---|---|---|---|---|
| Table legend: PDI - Polydispersity index, EE - Entrapment efficiency, FRR - Flow rate ratio, RLU - Relative luminescence units, RFU - Relative fluorescence units. | | | | | |

### Conclusion

LPNs with a higher content of PLGA (up to 60% w/w) can be formulated. The inventors found no differences in *FLuc* expression mediated by LPNs with lower (30% w/w) or higher (50% w/w) total lipids at doses ranging from 25 - 200 ng mRNA per well.

### Example 7 - Effect of PLGA type on physicochemical properties and in vitro performance

### Aim of study

The aim of this study was to investigate whether different PLGA types could be used in LPN formulations.

### Results

The final step in the optimisation process was to investigate the influence of PLGA type (**C33-C38, Table 2**) on the physicochemical properties and *in vitro* performance. Several PLGA types with different properties, *i.e.,* molecular weight, lactic/glycolic acid molar ratio, and type of end group modification (**Table 1**), were tested. LPNs were formulated using the NanoAssemblr^{®} Ignite microfluidic mixer. The process parameters were kept constant at a FRR of 10:1 and a TFR of 12 mL/min. The weight ratio between C12-200:*FLuc* mRNA was 10:1 with 2.5 mol% DMG-PEG₂₀₀₀. It was found that the PLGA type influenced the z-average, with no correlation between molecular weight and the end group termination. The z-averages were in the range of 150 - 200 nm, while the PDI was generally lower than 0.12 (**Figure 6A****, Table 7**)**.** However, there was a remarkable influence of PLGA type on *FLuc* expression in RAW 264.7 macrophages. LPNs formulated with P1 (50:50, 24-38 kDa, ester terminated) mediated a 2 to 3-fold higher expression than the other PLGA types (**Figure 6B**)**.**

**Table 7: Effect of PLGA type on physicochemical properties and in vitro performance**

| **Code** | **Size (nm)** | **PDI** | **EE (%)** | **FRR** | **Mean RLU/RFU (100 ng mRNA dose)** |
|---|---|---|---|---|---|
| **Effect of PLGA type (NanoAssemblr^{®} Ignite^{™}) ( data represent mean values ± SD, N = 2)** | | | | | |
| **C33** | 206 ± 13 | 0.12 ± 0.08 | 70 ± 4 | 10 | 6.4 × 10⁵ |
| **C34** | 211 ± 28 | 0.10 ± 0.03 | 67 ± 9 | 10 | 3.0 × 10⁵ |
| **C35** | 202 ± 5 | 0.05 ± 0.02 | 75 ± 3 | 10 | 3.6 × 10⁵ |
| **C36** | 159 ± 7 | 0.10 ± 0.02 | 54 ± 5 | 10 | 9.7 × 10⁴ |
| **C37** | 143 ± 14 | 0.05 ± 0.01 | 77 ± 5 | 10 | 1.1 × 10⁵ |
| **C38** | 177 ± 1 | 0.05 ± 0.02 | 68 ± 3 | 10 | 1.9 × 10⁵ |

| | | | | | |
|---|---|---|---|---|---|
| Table legend: PDI - Polydispersity index, EE - Entrapment efficiency, FRR - Flow rate ratio, RLU - Relative luminescence units, RFU - Relative fluorescence units. | | | | | |

### Conclusion

LPNs formulated with P1 (50:50, 24-38 kDa, ester terminated) mediated a higher luciferase expression than the other PLGA types. Different PLGA types have been assessed for LPN-mediated mRNA delivery by Zhao *et al.* (2018), who used the lipid TT3, and the transfection efficiency of mRNA was shown to increase when the hydrophobicity of PLGA was increased. This could be related to the results obtained here. As the ester-terminated PLGA is more hydrophobic, it should essentially give a higher response, which is evident from **P1** and **P3.** They both mediate higher luciferase expression than **P2** and **P4,** respectively. Also, acid-terminated PLGA is more prone to hydrolysis, which could also lead to a reduced stability and hence, lower response. When comparing **P5** and **P6,** the responses are generally higher for **P6** as it has a higher lactide content, which makes it more hydrophobic in nature.

### Example 8 - Effect of C12-200:mRNA weight ratio and total lipid content on endosomal escape

### Aim of study

The aim of this study was to investigate the effect of the C12-200:mRNA weight ratio and the total lipid content on the endosomal escape of the nucleic acids.

### Materials and methods

### Visualisation of Gal8 recruitment in HEK 293/T17 Gal8-GFP cells treated with LPNs and LNPs

The human embryonic kidney (HEK) 293/T17 Gal8-GFP reporter cell line was generated and cultured as reported previously (Herrera, M. *et al.* (2021)). Endosomal escape was quantified as Gal8 recruitment, which was visualised and quantified as previously reported with slight modifications (Herrera, M. *et al.* (2021)). First, µ-slide 8-well chamber slides (ibidi GmbH, Gräfelfing, Germany) were coated with poly-D-lysine (Thermo Fisher) overnight, and subsequently washed three times with 300 µL PBS. The HEK 293T/17 Gal8-GFP reporter cells were seeded in the coated chamber slides at a density of 4 × 10⁴ cells per well and allowed to adhere for 24 h. The cells were incubated with LPNs or control LNPs or Lipofectamine^{®} MessengerMAX^{™} at an mRNA dose of 200 ng, followed by washing with PBS, fixing with 4% (v/v) paraformaldehyde (Thermo Fisher), and staining with 4,6'-diamidino-2-phenylindole (DAPI, Thermo Fisher). The cells were imaged using a DMi8 inverted microscope (Leica Microsystems, Deerfield, IL, USA) equipped with an oil immersion objective at 40× magnification. An excitation band pass (BP) filter 470/40 nm with a dichromatic mirror 500 and suppression filter BP 525/50 nm were used to image the GFP signal, while the DAPI signal was detected using a BP 340-380 nm, 400 nm, and long pass 425 nm filter, respectively. Each formulation was tested in duplicate, and at least three fields of view per well were imaged blindly by an observer. The number of endosomal escape events per cell, detected as green puncta of Gal8 aggregates and blue nuclei, were quantified using the open-source image processing package Fiji (https://imagej.net/software/fiji/).

### Results

The inventors next investigated the ability of the LPNs to mediate endosomal escape. An array of LPNs, formulated by varying the C12-200:FLuc mRNA weight ratio and the total lipid content, was tested for the ability to escape the endosomes in the HEK 293T/17 reporter cell line. In theory, the LPNs disrupt endosomes thereby recruiting galectins in the HEK 293T/17 reporter cells, which would be observed as bright green dots under a confocal microscope due to the GFP fused to galectin-8. The GFP signal was normalised to the signal from the nucleus stained with DAPI relative to the signals from untreated (PBS) cells giving rise to the double-normalised puncta count. When the total lipid content was varied, a bell-shaped curve for the puncta count was observed, which closely resembles the *FLuc* expression (**Figure 7**). The mean *R²* values of a trendline drawn from 5:1 to 10:1 and 10:1 to 20:1 were 0.85 and 0.98 respectively. In contrast, no correlation was observed between the puncta count and the FRR, even though LPNs manufactured using FRRs of 1:1 and 3:1, respectively, mediated higher mRNA expression than LPNs prepared at other FRRs (data not shown).

### Conclusion

The highest degree of endosomal escape was observed for the LPNs with a total lipid content of 30% (w/w) relative to the PLGA polymer.

### Example 9 - LPNs mediate FLuc expression in vivo in mice

### Aim of study

During the previous examples, the physicochemical properties and *in vitro* performances of the LPNs were evaluated to optimise the LPN formulations. The aim of this study was therefore to test whether the LPNs were able to mediate *FLuc* expression *in vivo* in mice.

### Materials and methods

### In vivo bioluminescence imaging in mice

*In vivo* imaging was used to determine the biodistribution of protein (FLuc) expression upon subcutaneous administration of *FLuc* mRNA-loaded LPNs in mice. The experimental work was approved by the Danish National Experiment Inspectorate under permit 2022-15-0201-01221. The studies were performed in accordance with the European Community directive 86/609 for the care and use of laboratory animals. Female 8-10 weeks old BALB/cOlaHsd mice (ENVIGO, Limburg, Netherlands) were used for the experiments. The LPN formulations were administered by subcutaneous injection at the base of the tail near the right flank using a dose of 4 µg *FLuc* mRNA and a dose volume of 200 µL. Before imaging, the mice were anesthetised using isoflurane (up to 4% for induction, 1-3% for maintenance) and oxygen from a precision vaporiser, and D-luciferin (150 mg/kg) substrate was administered by intraperitoneal injection 20 min before imaging. Imaging was performed 6, 24, and 48 h after dosing using an In Vivo Imaging System (IVIS) Lumina XRMS imaging system (PerkinElmer, Waltham, MA, USA). Luminescence was measured independently of the exposure time as the total flux/s using the Living Image^{®} Software v4.7.4 (PerkinElmer).

### Results

Based on the *in vitro* studies, the inventors selected formulations with 30, 40, and 50% (w/w) total lipid content relative to PLGA, with 7 mol% DMPE-PEG₂₀₀₀ and a *C12-200:FLuc* mRNA weight ratio of 10:1. The inventors also wanted to test, if the FRR affects FLuc expression in mice. LPNs of specific compositions (**C39** - **C45, Table 2)** loaded with *FLuc* mRNA were formulated using the NanoAssemblr^{®} Ignite^{™} and injected subcutaneously in Balb/C mice. The FLuc expression in the mice was detected and quantified using an IVIS system. Physicochemical properties and *in vivo* performance of the formulations used for the biodistribution studies are shown in **Table 8.** A mean 100 % increase in luminescence was observed for 30% LPNs at 6 h prepared at a FRR of 10:1 compared to 30% LPNs prepared at a FRR of 3:1. 50% LPNs mediated a 57% increase in luminescence at 6 h relative to 30% LPNs when both were prepared at a FRR of 10:1. These LPNs mediated *FLuc* expression at the site of injection for at least 5 days (120 h) **(****Figure 8A****).** Representative images of the mice injected with 50% LPNs in IVIS are shown in **Figure 8B****.** The total luminescence flux at the site of injection at 6 h was significantly higher (*p* < 0.0001) for 30% LPNs prepared at a FRR of 3:1, compared to LPNs prepared at a FRR of 10:1 (**Figure 8C**).

**Table 8: Physicochemical properties and in vivo performance of the formulations used for biodistribution studies**

| **Code** | **Size (nm)** | **PDI** | **EE (%)** | **FRR** | **Mean Total flux (p/s) 6 h** |
|---|---|---|---|---|---|
| **Formulations injected for biodistribution studies in mice (NanoAssemblr^{®} Ignite^{™}) - DMPE-PEG₂₀₀₀ (N** = **1)** | | | | | |
| **C39** | 250 | 0.26 | 83 | 3 | 1.4 × 10⁷ |
| **C40** | 185 | 0.19 | 84 | 3 | 3.6 × 10⁷ |
| **C41** | 168 | 0.19 | 83 | 3 | 7.1 × 10⁷ |
| **C42** | 184 | 0.05 | 56 | 10 | 6.7 × 10⁶ |
| **C43** | 150 | 0.08 | 77 | 10 | 9.3 × 10⁷ |
| **C44** | 140 | 0.09 | 65 | 10 | 1.8 × 10⁸ |
| **C45** | 85 | 0.23 | 28 | 10 | - |

| | | | | | |
|---|---|---|---|---|---|
| Table legend: PDI - Polydispersity index, EE - Entrapment efficiency, FRR - Flow rate ratio. | | | | | |

### Conclusion

PLGA-LPNs comprising three different total lipid contents (30%, 40% and 50%) prepared at a FRR of 3:1 can mediate *FLuc* expression *in vivo* in mice. The LPNs prepared at a FRR of 10:1 elicit a higher total flux than the LPNs prepared at a FRR of 3:1, regardless of the total lipid content.

### Example 10 - High-resolution cryo-TEM shows the formation of multilayered lamellar structures along with polymeric particles

### Aim of study

The aim of this study was to investigate the structure of the LPNs using cryo-TEM.

### Materials and methods

*High-resolution cryogenic transmission electron microscopy (cryo-TEM)* Morphological analysis was carried out by cryo-TEM using a Titan Krios^{™} G2 transmission electron microscope [Field Electron and Ion Company (FEI), Hillsboro, OR, USA]. The samples were prepared using a FEI Vitrobot^{™} Mark IV by adding 2 µL formulation onto a Pelco Lacey carbon-filmed grid to form a thin film of approximately 10-500 nm. Subsequently, the grid was plunged into liquid ethane and transferred in liquid N₂ to an Oxford CT3500 cryo holder connected to the electron microscope. The samples were visualised in bright field mode at an acceleration voltage of 300 kV. Images were captured using a FEI Falcon^{™} direct electron detector (ThermoFisher Scientific).

### Results

High-resolution cryo-TEM analyses of the mRNA-loaded LPNs in their near-native state demonstrated the co-existence of equally sized multilamellar onion-like vesicles and spheres (**Figure 9**), which suggests that LPN components precipitate at different rates during the microfluidic manufacturing process, eventually resulting in structurally heterogeneous formulations. However, the heterogenous formulations were only observed when LPNs were formulated using a FRR of 10:1. This held true for 30% (**Figure 9B**) and 50% (w/w) LPNs (**Figure 9C**). When LPNs were formulated using a FRR of 3:1, a rather homogenous population was observed with lipids bilayers coating the surface of PLGA nanoparticles **(****Figure 9A****).**

### Conclusion

LPNs prepared at a FRR of 10:1 display a heterogeneous structure regardless of the total lipid content, whereas LPNs prepared at a FRR of 3:1 displayed a rather homogeneous structure with a dense electron core and lipid layers coating the PLGA, which might be evidence for the formation of LPNs. Differences in the mRNA transfection to cells arising due to LPNs formulated using different FRRs (Example 10) might be related to the nanostructure of LPNs. Structure has been shown to influence the mRNA expression *in vivo.* The presence of multiple bilayers or protrusions of bilayers from the PLGA core might be attributed to the total lipid content and the ejection of lipid layers from the PLGA when PBS is used as a buffer. Surprisingly, the homogenous, truly hybrid LPNs (Figure 9A) did not mediate *FLuc* expression as efficiently as the ones for which partial phase separation was observed.

### Example 11 - Immunogenicity

### Aim of study

The aim of this study was to investigate the immunogenicity of *OVA* mRNA-loaded LPNs.

### Materials and methods

### Immunisations

Seven to nine weeks-old female C57BL/6 mice (ENVIGO) were acquired and acclimatised for one week before experimental manipulation. Animals had free access to food and water. All experimental work was approved by the Danish National Experiment Inspectorate under permit 2022-15-0201-01203. The studies were performed in accordance with the European Community directive 86/609 for the care and use of laboratory animals. Mice were assigned to six groups of six individuals. All mice were immunised twice by subcutaneous administration at the base of the tail at an interval of 2 weeks using a dose volume of 200 µL PBS (pH 7.4). As a negative control, mice were primed and boosted with saline (n = 3). Another two groups of mice were immunised twice with the same dose of *OVA* mRNA entrapped in LPNs comprising 30 and 50% total lipids relative to PLGA. As positive control, mice were immunised twice intraperitoneally with 5 µg endograde chicken egg OVA adjuvanted with CAF^{®}09b (Kosholm, KS. *et al.* (2014)), which is a liposome-based adjuvant composed of dioctadecylammonium (DDA) bromide, monomycoloyl glycerol (MMG) and polyinosinic:polycytidylic acid [poly(I:C)] known to induce strong CD8⁺ T-cell responses. CAF^{®}09b was administered at a dose of 250/50/12.5 µg DDA/MMG/poly(I:C).

### Sample collection and cell preparation

At 4 weeks of the study, *i.e.,* two weeks after the booster immunisation, animals were euthanised. Blood was collected by cardiac puncture, and serum was separated by spontaneous clotting at RT and extracted by centrifugation (2000 × g, for 10 min) using a Heraeus Multifuge 3SR+ (Thermo Fischer Scientific). Serum was stored at -20 °C until antibody detection. Spleens were aseptically harvested from the euthanised mice. The spleens were homogenised using a 70 µm nylon mesh cell-strainer (Falcon, Durham, NC, USA), and washed twice with PBS to obtain single-cell suspensions. Spleen cells were then grown in 96-well microtiter plates (Nunc, Roskilde, Denmark) containing 2 × 10⁵ cells per well for cytokine assays, and 1 × 10⁶ cells per well for flow cytometry, respectively, in 100 µL RPMI-1640 (Sigma-Aldrich) supplemented with 5 × 10⁻⁵ M 2-mercaptoethanol (Gibco Thermo Fisher), 1% (v/v) sodium pyruvate (Sigma-Aldrich), 1% (v/v) penicillin-streptomycin (Gibco Thermo Fisher), 1% HEPES (Gibco Thermo Fisher), and 10% (v/v) fetal calf serum (FCS, Gibco Thermo Fisher).

### Flow cytometry

Splenocytes were stimulated with full-length OVA (5 µg/mL), OVA₂₅₇₋₂₆₄ (SIINFEKL) (SEQ ID NO.: 6) and OVA₃₂₃₋₃₃₉ peptide (ISQAVHAAHAEINEAGR) (SEQ ID NO.: 7) (both 5 µg/mL, AnaSpec, Fremont, CA, USA), respectively, or Concanavalin A (5 µg/mL, Sigma Aldrich) containing anti-CD28 (37.51) and anti-CD49d (9C10) costimuli (both 1 µg/mL, BD Biosciences) at 37 °C, 5% CO₂ for 12 h, with brefeldin A (10 µg/mL, Sigma-Aldrich) and monensin/Golgi-stop (0.7 µL/mL, BD Bioscience) added during the last 11 h of incubation. For detection of polyfunctional epitope-specific CD4⁺ T cells, a previously described protocol was used that combines MHC-II tetramer and intracellular cytokine staining (ICS) (Ciabattini, A. *et al.* (2015)). Based on this protocol, the detection of polyfunctional epitope-specific CD8⁺ T cells was optimised by combining MHC-I pentamer staining with ICS. Following overnight storage at 4 °C, the spleen cells were labelled with Fixable Viability Stain (FVS) 510 (BD Biosciences, 1:1,000, 100 µL/well) for 20 min at 4 °C in the dark and washed twice with PBS. Cells were fixed and permeabilised for 20 min at 4 °C with BD Cytofix/Cytoperm (BD Biosciences). Samples were then blocked for 30 min at 4 °C in Fc-blocking solution (5 µg/mL of CD16/CD32 mAb, BD Bioscience) and stained for 1 h at RT with PE-conjugated H-2Kb-SIINFEKL (OVA₂₅₇₋₂₆₄) (MHC-I) pentamer (diluted 1:8, ProImmune, Oxford, UK) or PE-conjugated I-A^{b}-ISQAVHAAHAEINEAGR (OVA₃₂₃₋₃₃₉) (MHC-II) tetramer (diluted 1:8, ProImmune) diluted in perm/wash buffer. In the final 20 min of tetramer incubation, the following mix of fluorescent antibodies were added: anti-CD4-BUV395 (RM4-5; BD Biosciences), anti-CD8-BUV737 (53-6.7; BD Biosciences), and anti-CD44-FITC (IM7; BD Biosciences). All cells were washed twice, resuspended in FACS buffer, and analysed using an LSRFortessa flow cytometer (BD Biosciences). Gates for the surface markers are based on fluorescence-minus-one controls. The gating strategy used to identify distinct cell populations in the spleen is based on previous reports (Vono, M. *et al.* (2019), Christensen, D. *et al.* (2017), Thakur, A. *et al.* (2018)). All flow cytometric data analyses were performed using the FlowJo software v10 (Tree Star, Ashland, OR, USA).

### Results

To evaluate the immunogenicity of *OVA* mRNA-loaded LPNs, mice were vaccinated twice by subcutaneous prime and boost immunisation at 2 weeks interval. Immune responses were assessed by *ex vivo* re-stimulation of isolated splenocytes with antigen, followed by tetramer staining and quantification of phenotypic marker expression (**Figure 10**). Immunisation with *OVA* mRNA-loaded C12-200 LPNs (**C47, C48, Table 2 and Table 9**) resulted in induction of OVA₂₅₇₋₂₆₄-specific MHC-I (SIINFEKL) Tet⁺CD8⁺CD44⁺ T cells in the spleen, but the percentages were not significantly higher than the responses measured for the negative control C12-200 LPNs (**C46**) loaded with eGFP mRNA. For the LPNs modified with 50% lipid (**C47**)**,** there was no statistically significant difference in the antigen-specific CD8⁺ T-cell responses in the spleen between a dose of 5 µg and 10 µg *OVA* mRNA (**Figure 10**). In addition, there was no statistically significant difference in the antigen-specific CD8⁺ T-cell responses in the spleen induced by LPNs modified with 30% (**C48**) and 50% lipid (**C47**), respectively (**Figure 10**)**.** Intraperitoneal prime-boost vaccination with the subunit vaccine consisting of OVA adjuvanted CAF^{®}09b induced approximately 5% OVA₂₅₇₋₂₆₄-specific MHC-I (SIINFEKL) Tet⁺CD8⁺CD44⁺ T cells in the spleen (**Figure 10**), but this percentage of OVA₂₅₇₋₂₆₄-specific MHC-I (SIINFEKL) Tet⁺CD8⁺CD44⁺ T cells was not significantly different from the negative controls.

**Table 9: Physicochemical properties and in vivo performance of the formulations used for vaccination studies in mice**

| **Code** | **Size (nm)** | **PDI** | **EE (%)** | **FRR** | **Mean Total flux (p/s) 6 h** |
|---|---|---|---|---|---|
| **Formulations used for vaccination studies in mice (NanoAssemblr^{®} Ignite^{™}) - DMPE PEG₂₀₀₀ (N = 2)** | | | | | |
| **C46 (*eGFP*)** | 167 ± 24 | 0.06 | 43 ± 24 | 10 | - |
| **C47 (*OVA*)** | 189 ± 21 | 0.08 | 47 ± 9 | 10 | - |
| **C48 (*OVA*)** | 255 ± 32 | 0.13 | 38 ± 25 | 10 | - |

| | | | | | |
|---|---|---|---|---|---|
| Table legend: PDI - Polydispersity index, EE - Entrapment efficiency, FRR - Flow rate ratio. | | | | | |

### Conclusion

Immunisation with *OVA* mRNA-loaded C12-200 LPNs resulted in induction of OVA₂₅₇₋₂₆₄-specific MHC-I (SIINFEKL) Tet⁺CD8⁺CD44⁺ T cells in the spleen, but the percentages were not significantly higher than the responses measured for the negative control C12-200 LPNs loaded with eGFP mRNA.

### References

- WO 2017/158093 A1
- Zhao, W. et al.: Lipid Polymer Hybrid Nanomaterials for mRNA Delivery, Cell Mol Bioeng, 2018 Oct;11(5):397-406
- Love KT, Mahon KP, Levins CG, Whitehead KA, Querbes W, Dorkin JR, et al. Lipid-like materials for low-dose, in vivo gene silencing. Proceedings of the National Academy of Sciences. 2010;107(5):1864-9
- Thanki K, Zeng X, Justesen S, Tejlmann S, Falkenberg E, Van Driessche E, et al. Engineering of small interfering RNA-loaded lipidoid-poly(DL-lactic-co-glycolic acid) hybrid nanoparticles for highly efficient and safe gene silencing: A quality by design-based approach. European Journal of Pharmaceutics and Biopharmaceutics. 2017;120:22-33
- Kauffman KJ, Dorkin JR, Yang JH, Heartlein MW, DeRosa F, Mir FF, et al. Optimization of Lipid Nanoparticle Formulations for mRNA Delivery in Vivo with Fractional Factorial and Definitive Screening Designs. Nano Letters. 2015; 15(11): 7300-6.
- Kauffman KJ, Mir FF, Jhunjhunwala S, Kaczmarek JC, Hurtado JE, Yang JH, et al. Efficacy and immunogenicity of unmodified and pseudouridine-modified mRNA delivered systemically with lipid nanoparticles in vivo. Biomaterials. 2016;109:78-87
- Lokras A, Chakravarty A, Rades T, Christensen D, Franzyk H, Thakur A, et al. Simultaneous quantification of multiple RNA cargos co-loaded into nanoparticle-based delivery systems. International Journal of Pharmaceutics. 2022;626:122171
- Lokras A, Thakur A, Wadhwa A, Thanki K, Franzyk H, Foged C. Optimizing the Intracellular Delivery of Therapeutic Anti-inflammatory TNF-o siRNA to Activated Macrophages Using Lipidoid-Polymer Hybrid Nanoparticles. Frontiers in Bioengineering and Biotechnology. 2021;8.
- Herrera M, Kim J, Eygeris Y, Jozic A, Sahay G. Illuminating endosomal escape of polymorphic lipid nanoparticles that boost mRNA delivery. Biomaterials Science. 2021;9(12):4289-300.
- Korsholm KS, Hansen J, Karlsen K, Filskov J, Mikkelsen M, Lindenstrøm T, et al. Induction of CD8+ T-cell responses against subunit antigens by the novel cationic liposomal CAF09 adjuvant. Vaccine. 2014;32(31):3927-35.
- Ciabattini A, Prota G, Christensen D, Andersen P, Pozzi G, Medaglini D. Characterization of the Antigen-Specific CD4+ T Cell Response Induced by Prime-Boost Strategies with CAF01 and CpG Adjuvants Administered by the Intranasal and Subcutaneous Routes. Frontiers in Immunology. 2015;6.
- Vono, M., Eberhardt, C.S., Auderset, F., Mastelic-Gavillet, B., Lemeille, S., Christensen, D., Andersen, P., Lambert, P.H. and Siegrist, C.A. (2019) Maternal Antibodies Inhibit Neonatal and Infant Responses to Vaccination by Shaping the Early-Life B Cell Repertoire within Germinal Centers. Cell Rep, 28, 1773-1784 e1775.
- Christensen, D., Mortensen, R., Rosenkrands, I., Dietrich, J. and Andersen, P. (2017) Vaccine-induced Th17 cells are established as resident memory cells in the lung and promote local IgA responses. Mucosal Immunol, 10, 260-270.
- Thakur, A., Rodriguez-Rodriguez, C., Saatchi, K., Rose, F., Esposito, T., Nosrati, Z., Andersen, P., Christensen, D., Hafeli, U.O. and Foged, C. (2018) Dual-Isotope SPECT/CT Imaging of the Tuberculosis Subunit Vaccine H56/CAF01: Induction of Strong Systemic and Mucosal IgA and T-Cell Responses in Mice Upon Subcutaneous Prime and Intrapulmonary Boost Immunization. Front Immunol, 9, 2825.

### Sequence listing

| SEQ ID NO. | Sequence name | Sequence* |
|---|---|---|
| 1 | *FLuc* mRNA ORF | *See sequence listing* |
| 2 | *OVA* mRNA ORF | *See sequence listing* |
| 3 | siRNA-TNF-α sense sequence | 5'-pGUCUCAGCCUCUUCUCAUUCCUGct-3' |
| 4 | siRNA-TNF-α antisense sequence | 5'- AGCAGGAAUGAGAAGAGGCUGAGACAU-3' |
| 5 | SARS-CoV2 spike protein | *See sequence listing* |
| 6 | OVA₂₅₇₋₂₆₄ | SIINFEKL |
| 7 | OVA₃₂₃₋₃₃₉ | ISQAVHAAHAEINEAGR |
| 8 | ESAT-6 | *See sequence listing* |
| 9 | Secreted antigen 85b (Ag85B) | *See sequence listing* |
| 10 | Plasmodium falciparum 3D7 circumsporozoite | *See sequence listing* |
| 11 | RSV-F Long mRNA | *See sequence listing* |

| | | |
|---|---|---|
| * Lower case letters represent deoxyribonucleotides and *p* represents phosphate residues. | | |

## Claims

1. A nanoparticle composition comprising a cationic or cationically ionisable lipid or lipid-like material, a helper lipid, a lipopolymer, and one or more variants of poly(D,L-lactic-*co*-glycolic acid) (PLGA),
wherein the cationic or cationically ionisable lipid or lipid-like material is selected from the group consisting of 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3'',3'''-(ethane-1,2-diylbis(azanetriyl))tetrakis(N-(2-((2-hydroxytetradecyl)amino)ethyl)propanamide) (G0-C14), N1,N3,N5-tris(3-(didodecylamino)propyl)benzene-1,3,5-tricarboxamide (TT3), dilinoleylmethyl-4-dimethylaminobutyrate (DLin-MC3-DMA), 2,2-dilinoleyl-4-dimethylaminoethyl-[1,3]-dioxolane (DLin-KC2-DMA), hexa(octan-3-yl) 9,9',9",9‴,9‴′,9‴ʺ- ((((benzene-1,3,5-tricarbonyl)yris(azanediyl)) tris (propane-3,1-diyl)) tris(azanetriyl))hexanonanoate (FTT5), 1,2-dioleoyl-3-trimethylamonniumpropane (DOTAP), dimethyldioctadecylammonium bromide (DDAB), 1,2-dioleoyl-3-dimethylammonium-chloride (DODAC), and 1,2-di-*O-*octadecenyl-3-trimethylammonium propane (DOTMA), or any mixture thereof,
wherein the helper lipid is selected from the group consisting of 1,2-dioleoyl-*sn-*glycero-3-phosphoethanolamine (DOPE), 1,2-dioleoyl-*sn*-glycero-3-phosphocholine (DOPC), 1,2-dipalmitoyl-sn-glycero-3-phosphocholine (DPPC), 1,2-distearoyl-*sn*-glycero-3-phosphocholine (DSPC), and 1,2-ditetradecanoyl-*sn-*glycero-3-phosphocholine (DMPC), or any mixture thereof, and
wherein the lipopolymer is a polyethylene glycol (PEG)- or polysarcosine-lipid conjugate, or a PEG- or polysarcosine-lipid like conjugate, or any mixture thereof.

2. The nanoparticle composition according to claim 1, wherein the lipopolymer is selected from the group consisting of 1,2-dimyristoyl-*sn*-glycero-3-phosphoethanolamine-N-[methoxy(polyethylene glycol)-2000] (DMPE-PEG₂₀₀₀), 1,2-dimyristoyl-*rac*-glycero-3-methoxypolyethylene glycol-2000 (DMG-PEG₂₀₀₀), 1,2-distearoyl-*sn*-glycero-3-phosphoethanolamine-N-[amino(polyethylene glycol)-2000] DSPE-PEG₂₀₀₀, 2-[(polyethylene glycol)-2000]-N,N-ditetradecylacetamide (ALC-0159), N-(methylpolyoxyethylene oxycarbonyl)-1,2-dipalmitoyl-*sn*-glycero-3-phosphoethanolamine (DPPE-PEG), 1,2-distearoyl-*rac*-glycerol-3-methoxypolyethylene glycol (DSG-PEG), N-tetradecyl polysarcosine25, N-hexadecyl polysarcosine25, N-octadecyl polysarcosine25, N-dodecyl polysarcosine25, N,N-ditetradecylamine-N-succinyl[methyl(polysarcosine)45], N,N-ditetradecylamine-N-succinyl[methyl(polysarcosine)35], and N,N-ditetradecyl-polysarcosine-25, or any mixture thereof.

3. The nanoparticle composition according to any one of claims 1 or 2, wherein the cationic or cationically ionisable lipid or lipid-like material is 1 ,1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), tetrakis(8-methylnonyl) 3,3',3",3‴-(((methylazanediyl)bis(propane-3,1-diyl))bis(azanetriyl))tetrapropionate (306Oi10), 1,2-dioleoyl-3-trimethylamonniumpropane (DOTAP), dimethyldioctadecylammonium bromide (DDAB), 1,2-dioleoyl-3-dimethylammonium-chloride (DODAC), or 1,2-di-*O-*octadecenyl-3-trimethylammonium propane (DOTMA), preferably C12-200.

4. The nanoparticle composition according to any one of the preceding claims, wherein the helper lipid is 1,2-dioleoyl-*sn*-glycero-3-phosphocholine (DOPC), or 1,2-dioleoyl-*sn*-glycero-3-phosphoethanolamine (DOPE), preferably DOPE.

5. The nanoparticle composition according to any one of the preceding claims, wherein the lipopolymer is 1,2-dimyristoyl-*sn*-glycero-3-phosphoethanolamine-N-[methoxy(polyethylene glycol)-2000] (DMPE-PEG₂₀₀₀), N-(methylpolyoxyethylene oxycarbonyl)-1,2-dipalmitoyl-*sn*-glycero-3-phosphoethanolamine (DPPE-PEG), 1,2-distearoyl-*rac*-glycerol-3-methoxypolyethylene glycol (DSG-PEG), N-tetradecyl polysarcosine25, 1,2-distearoyl-*sn*-glycero-3-phosphoethanolamine-N-[amino(polyethylene glycol)-2000] (DSPE-PEG₂₀₀₀), or 1,2-distearoyl-*sn*-glycero-3-phosphoethanolamine-N-[amino(polyethylene glycol)-2000] (DMG-PEG₂₀₀₀), preferably DMPE-PEG₂₀₀₀ or DMG-PEG₂₀₀₀.

6. The nanoparticle composition according to any one of the preceding claims, wherein the one or more variants of poly(D,L-lactic-co-glycolic acid) (PLGA) have at least one ester-end group modification, or at least one acid-end group modification, preferably at least one ester-end group modification.

7. The nanoparticle composition according to any one of the preceding claims, wherein said nanoparticle composition does not comprise cholesterol.

8. The nanoparticle composition according to any one of the preceding claims, wherein the nanoparticle composition further comprises at least one nucleic acid.

9. The nanoparticle composition according to claim 8, wherein the at least one nucleic acid is selected from the group consisting of messenger RNA (mRNA), selfamplifying RNA, circular RNA (circRNA), plasmid DNA (pDNA), small interfering RNA (siRNA), single guide RNA (sgRNA), guide RNA (gRNA), long non-coding RNA (lncRNA), small activating RNA (saRNA), and splice-switching antisense oligonucleotide (ASO).

10. A vaccine composition comprising the nanoparticle composition according to any one of the preceding claims and at least one nucleic acid encoding an antigen.

11. The vaccine composition according to claim 10, wherein the antigen is selected from the group consisting of corona virus antigens, such as SARS-CoV and MERS-CoV antigens, such as SARS-CoV2 spike protein (SEQ ID NO.: 5) or receptor binding domain (RBD), *Mycobacterium tuberculosis* antigens (SEQ ID NO.: 8-9), *Plasmodium falciparum* antigens (SEQ ID NO.: 10), respiratory syncytial virus (RSV) antigens (SEQ ID NO.: 11), Ebolavirus antigens, Marburg virus antigens, Lassa virus antigens, Nipah virus antigens, Zika virus antigens, Crimean-Congo haemorrhagic fever orthonairovirus antigens, human papilloma virus (HPV) antigens, and influenza antigens.

12. The vaccine composition according to any one of claims 10 or 11 for use in the prevention and/or treatment of an infectious disease.

13. A process for obtaining the nanoparticle composition according to any one of claims 1-x, said process comprising the steps of:
a) Providing an cationic or cationically ionisable lipid or lipid-like material, a helper lipid, a lipopolymer, one or more variants of poly(D,L-lactic-co-glycolic acid) (PLGA), and at least one nucleic acid;
b) Dissolving the cationic or cationically ionisable lipid or lipid-like material, the helper lipid, the lipopolymer, and the one or more variants of PLGA of step a) in an organic solvent comprising acetonitrile supplemented with acetic acid, thereby providing an organic phase;
c) Diluting the at least one nucleic acid of step a) in an aqueous solvent comprising a buffer with a pH within the range of 3.0 to 7.8, thereby providing an aqueous phase;
d) Mixing the organic phase of step b) with the aqueous phase of step c) to obtain nanoparticles by nanoprecipitation;
e) Performing filtration, preferably tangential flow filtration or dialysis, of the nanoparticles of step d) to obtain a nanoparticle composition;
wherein the cationic or cationically ionisable lipid or lipid-like material is selected from the group consisting of 1 '-((2-(4-(2-((2-(bis(2-hydroxydodecyl)amino)ethyl)(2-hydroxydodecyl)amino)ethyl) piperazin-1-yl)ethyl)azanediyl)bis(dodecan-2-ol) (C12-200), N1,N16-didodecyl-4,7,13-tris[3-(dodecylamino)-3-oxopropyl]-4,7,10,13-tetraazahexadecanediamide (98N12-5), tetrakis(8-methylnonyl) 3,3',3",3‴-(((methylazanediyl)bis(propane-3,1-diyl))bis(azanetriyl))tetrapropionate (306Oi10), 3,3',3",3‴-(ethane-1,2-diylbis(azanetriyl))tetrakis(N-(2-((2-hydroxytetradecyl)amino)ethyl)propanamide) (G0-C14), dilinoleylmethyl-4-dimethylaminobutyrate (DLin-MC3-DMA), N1,N3,N5-tris(3-(didodecylamino)propyl)benzene-1,3,5-tricarboxamide (TT3), 2,2-dilinoleyl-4-dimethylaminoethyl-[1,3]-dioxolane (DLin-KC2-DMA), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), and 1,2-dioleoyl-3-trimethylamonniumpropane (DOTAP), or any mixture thereof,
wherein the helper lipid is selected from the group consisting of 1,2-dioleoyl-sn-glycero-3-phosphoethanolamine (DOPE), DOPC, DOPG, DPPC, DSPC, DPPG, DMPC, MGDG, DGDG, and SQDG, or any mixture thereof, and
wherein the lipopolymer is a PEG- or polysarcosine-lipid conjugate, or a PEG- or polysarcosine-lipid like conjugate, or any mixture thereof.

14. The process according to claim 13, wherein the acetonitrile of step b) is supplemented with acetic acid in a concentration in the range of 1.0% to 10% (v/v), such as 1.5% to 8.0% (v/v), such as 2.0% to 5.0% (v/v), preferably 5.0% (v/v).

15. A nanoparticle composition obtained using the process of any one of claims 13 or 14.
